# EUROPEAN PATENT SPECIFICATION

(11) **EP 0 667 911 B1**
(45) Date of publication and mention of the grant of the patent: **06.05.1999**
(21) Application number: 93922753.4
(22) Date of filing: 22.09.1993
(51) Int. Cl.: C12N 15/85, C12N 9/02, C12N 15/53, G01N 33/50

(54) **STABLY-TRANSFORMED MAMMALIAN CELLS EXPRESSING A REGULATED, INFLAMMATORY CYCLOOXYGENASE**
STABIL TRANSFORMIERTE SÄUGERTIERZELLEN, DIE EINE GEREGELTE, ENTZÜNDLICHE CYCLOOXYGENASE EXPRIMIEREN
CELLULES DE MAMMIFERES A TRANSFORMATION STABLE EXPRIMANT UNE CYCLOOXYGENASE INFLAMMATOIRE REGULEE

(30) Priority: 22.09.1992 US 949780; 01.12.1992 US 983835; 22.03.1993 US 34143; 28.04.1993 US 54364
(43) Date of publication of application: 23.08.1995
(73) Proprietor: University of Rochester, Rochester, New York 14627 (US)
(72) Inventor: YOUNG, Donald, A., Rochester, NY 14618 (US); O'BANION, Michael, K., Pittsford, NY 14534 (US); WINN, Virginia, D., Rochester, NY 14620 (US)
(74) Representative: Matthews, Derek Peter
(86) International application number: US9309167
(87) International publication number: WO9406919

(56) References cited:
- PROCEEDINGS OF THE NATIONAL ACADEMY OF SCIENCES OF USA. vol. 89, no. 16 , 15 August 1992 , WASHINGTON US pages 7384 - 7388 TIMOTHY HLA ET AL. 'Human cyclooxygenase-2 cDNA'
- PROCEEDINGS OF THE NATIONAL ACADEMY OF SCIENCES OF USA. vol. 89 , June 1992 , WASHINGTON US pages 4888 - 4892 M. KERRY O'BANION ET AL. 'cDNA cloning and functional activity of a glucocorticoid-regulated inflammatory cyclooxygenase'
- PROSTAGLANDINS, LEUKOTRIENES, LIPOXINS, AND PAF 1991 pages 67 - 78 DANIEL L. SIMMONS ET AL. 'Multiple cyclooxygenases: cloning of a mitogen-inducible form'
- CELL GROWTH & DIFFERENTIATION vol. 3, no. 7 , July 1992 pages 443 - 450 R.P. RYSECK ET AL. 'Identification of an immediate early gene, pghs-B, whose protein product has prostaglandin synthase/cyclooxygenase activity'
- FASEB JOURNAL vol. 5, no. 9 , June 1991 , BETHESDA, MD US pages 2304 - 2312 COLIN D. FUNK ET AL. 'Human platelet/erythroleukemia cell porstaglandin G/H synthase: cDNA cloning, expression, and gene chromosomal assignment'
- JOURNAL OF LIPID MEDIATORS vol. 6, no. 1-3 , March 1993 pages 119 - 129 ELIZABETH A. MEADE ET AL. 'Expression of the murine prostaglandin (PGH) synthase-1 and PGH synthase-2 isozymes in cos-1 cells'
- THE AMERICAN JOURNAL OF MEDICINE vol. 95, no. 2A , 9 August 1993 pages 2A40S - 2A44S DAVID L. DEWITT ET AL. 'PGH synthase isoenzyme selectivity: The potential for safer nonsteroidal antiinflammatory drugs'
- JOURNAL OF BIOLOGICAL CHEMISTRY vol. 268, no. 9 , 25 March 1993 , BALTIMORE, MD US pages 6610 - 6614 ELIZABETH A. MEADE ET AL. 'Differential inhibition of prostaglandin endoperoxide synthase (cyclooxygenase) isozymes by aspirin and other non-steroidal antiinflammatory drugs'

### SEQUENCE LISTING

### (1) GENERAL INFORMATION:

(i) APPLICANT:
   Young, Donald A.
   O'Banion, M. Kerry
   Winn, Virginia D.
(ii) TITLE OF INVENTION: Stably-Transformed Mammalian Cells Expressing a Regulated, Inflammatory Cyclooxygenase
(iii) NUMBER OF SEQUENCES: 13
(iv) CORRESPONDENCE ADDRESS:
   (A) ADDRESSEE: Merchant & Gould
   (B) STREET: 3100 Norwest Center
   (C) CITY: Minneapolis
   (D) STATE: MN
   (E) COUNTRY: USA
   (F) ZIP: 55402
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER:
   (B) FILING DATE:
   (C) CLASSIFICATION:
(viii) ATTORNEY/AGENT INFORMATION:
   (A) NAME: Woessner, Warren D.
   (B) REGISTRATION NUMBER: 30,440
   (C) REFERENCE/DOCKET NUMBER: 8840.20-US-01
(ix) TELECOMMUNICATION INFORMATION:
   (A) TELEPHONE: 612-332-5300
   (B) TELEFAX: 612-332-9081

### (2) INFORMATION FOR SEQ ID NO:1:

(i) SEQUENCE CHARACTERISTICS:
   (A) LENGTH: 1920 base pairs
   (B) TYPE: nucleic acid
   (C) STRANDEDNESS: single
   (D) TOPOLOGY: linear
(ii) MOLECULE TYPE: cDNA
(vi) ORIGINAL SOURCE:
   (A) ORGANISM: Murine gri PGHS
(xi) SEQUENCE DESCRIPTION: SEQ ID NO:1:
   CTTCAGGAGT CAGTCAGGAC TCTGCTCACG AAGGAACTCA GCACTGCATC CTGCCAGCTC     60
   CACCGCCACC ACTACTGCCA CCTCCGCTGC CACCTCTGCG ATGCTCTTCC GAGCTGTGCT    120
   GCTCTGCGCT GCCCTGGGGC TCAGCCAGGC AGCAAATCCT TGCTGTTCCA ATCCATGTCA    180
   AAACCGTGGG GAATGTATGA GCACAGGATT TGACCAGTAT AAGTGTGACT GTACCCGGAC    240
   TGGATTCTAT GGTGAAAACT GTACTACACC TGAATTTCTG ACAAGAATCA AATTACTGCT    300
   GAAGCCCACC CCAAACACAG TGCACTACAT CCTGACCCAC TTCAAGGGAG TCTGGAACAT    360
   TGTGAACAAC ATCCCCTTCC TGCGAAGTTT AATCATGAAA TATGTGCTGA CATCCAGATC    420
   ATATTTGATT GACAGTCCAC CTACTTACAA TGTGCACTAT GGTTACAAAA GCTGGGAAGC    480
   CTTCTCCAAC CTCTCCTACT ACACCAGGGC CCTTCCTCCC GTAGCAGATG ACTGCCCAAC    540
   TCCCATGGGT GTGAAGGGAA ATAAGGAGCT TCCTGATTCA AAAGAAGTGC TGGAAAAGGT    600
   TCTTCTACGG AGAGAGTTCA TCCCTGACCC CCAAGGCTCA AATATGATGT TTGCATTCTT    660
   TGCCCAGCAC TTCACCCATC AGTTTTTCAA GACAGATCAT AAGCGAGGAC CTGGGTTCAC    720
   CCGAGGACTG GGCCATGGAG TGGACTTAAA TCACATTTAT GGTGAAACTC TGGACAGACA    780
   ACATAAACTG CGCCTTTTCA AGGATGGAAA ATTGAAATAT CAGGTCATTG GTGGAGAGGT    840
   GTATCCCCCC ACAGTCAAAG ACACTCAGGT AGAGATGATC TACCCTCCTC ACATCCCTGA    900
   GAACCTGCAG TTTGCTGTGG GGCAGGAAGT CTTTGGTCTG GTGCCTGGTC TGATGATGTA    960
   TGCCACCATC TGGCTTCGGG AGCACAACAG AGTGTGCGAC ATACTCAAGC AGGAGCATCC   1020
   TGAGTGGGGT GATGAGCAAC TATTCCAAAC CAGCAGACTC ATACTCATAG GAGAGACTAT   1080
   CAAGATAGTG ATCGAAGACT ACGTGCAACA CCTGAGCGGT TACCACTTCA AACTCAAGTT 1140
   TGACCCAGAG CTCCTTTTCA ACCAGCAGTT CCAGTATCAG AACCGCATTG CCTCTGAATT 1200
   CAACACACTC TATCACTGGC ACCCCCTGCT GCCCGACACC TTCAACATTG AAGACCAGGA 1260
   GTACAGCTTT AAACAGTTTC TCTACAACAA CTCCATCCTC CTGGAACATG GACTCACTCA 1320
   GTTTGTTGAG TCATTCACCA GACAGATTGC TGGCCGGGTT GCTGGGGGAA GAAATGTGCC 1380
   AATTGCTGTA CAAGCAGTGG CAAAGGCCTC CATTGACCAG AGCAGAGAGA TGAAATACCA 1440
   GTCTCTCAAT GAGTACCGGA AACGCTTCTC CCTGAAGCCG TACACATCAT TTGAAGAACT 1500
   TACAGGAGAG AAGGAAATGG CTGCAGAATT GAAAGCCCTC TACAGTGACA TCGATGTCAT 1560
   GGAACTGTAC CCTGCCCTGC TGGTGGAAAA ACCTCGTCCA GATGCTATCT TTGGGGAGAC 1620
   CATGGTAGAG CTTGGAGCAC CATTCTCCTT GAAAGGACTT ATGGGAAATC CCATCTGTTC 1680
   TCCTCAATAC TGGAAGCCGA GCACCTTTGG AGGCGAAGTG GGTTTTAAGA TCATCAATAC 1740
   TGCCTCAATT CAGTCTCTCA TCTGCAATAA TGTGAAGGGG TGTCCCTTCA CTTCTTTCAA 1800
   TGTGCAAGAT CCACAGCCTA CCAAAACAGC CACCATCAAT GCAAGTGCCT CCCACTCCAG 1860
   ACTAGATGAC ATTAACCCTA CAGTACTAAT CAAAAGGCGT TCAACTGAGC TGTAAAAGTC 1920

### (2) INFORMATION FOR SEQ ID NO:2:

(i) SEQUENCE CHARACTERISTICS:
   (A) LENGTH: 604 amino acids
   (B) TYPE: amino acid
   (C) STRANDEDNESS: single
   (D) TOPOLOGY: linear
(ii) MOLECULE TYPE: protein
(vi) ORIGINAL SOURCE:
   (A) ORGANISM: Amino acid sequence for Murine gri PGHS
(xi) SEQUENCE DESCRIPTION: SEQ ID NO:2:
   Met Leu Phe Arg Ala Val Leu Leu Cys Ala Ala Leu Gly Leu Ser Gln
   1               5                   10                  15
   Ala Ala Asn Pro Cys Cys Ser Asn Pro Cys Gln Asn Arg Gly Glu Cys
               20                  25                  30
   Met Ser Thr Gly Phe Asp Gln Tyr Lys Cys Asp Cys Thr Arg Thr Gly
           35                  40                  45
   Phe Tyr Gly Glu Asn Cys Thr Thr Pro Glu Phe Leu Thr Arg Ile Lys
       50                  55                  60
   Leu Leu Leu Lys Pro Thr Pro Asn Thr Val His Tyr Ile Leu Thr His
   65                  70                  75                  80
   Phe Lys Gly Val Trp Asn Ile Val Asn Asn Ile Pro Phe Leu Arg Ser
                   85                  90                  95
   Leu Ile Met Lys Tyr Val Leu Thr Ser Arg Ser Tyr Leu Ile Asp Ser
               100                 105                 110
   Pro Pro Thr Tyr Asn Val His Tyr Gly Tyr Lys Ser Trp Glu Ala Phe
           115                 120                 125
   Ser Asn Leu Ser Tyr Tyr Thr Arg Ala Leu Pro Pro Val Ala Asp Asp
       130                 135                 140
   Cys Pro Thr Pro Met Gly Val Lys Gly Asn Lys Glu Leu Pro Asp Ser
   145                 150                 155                 160
   Lys Glu Val Leu Glu Lys Val Leu Leu Arg Arg Glu Phe Ile Pro Asp
                   165                 170                 175
   Pro Gln Gly Ser Asn Met Met Phe Ala Phe Phe Ala Gln His Phe Thr
               180                 185                 190
   His Gln Phe Phe Lys Thr Asp His Lys Arg Gly Pro Gly Phe Thr Arg
   195 200 205
   Gly Leu Gly His Gly Val Asp Leu Asn His Ile Tyr Gly Glu Thr Leu
   210 215 220
   Asp Arg Gln His Lys Leu Arg Leu Phe Lys Asp Gly Lys Leu Lys Tyr
   225 230 235 240
   Gln Val Ile Gly Gly Glu Val Tyr Pro Pro Thr Val Lys Asp Thr Gln
   245 250 255
   Val Glu Met Ile Tyr Pro Pro His Ile Pro Glu Asn Leu Gln Phe Ala
   260 265 270
   Val Gly Gln Glu Val Phe Gly Leu Val Pro Gly Leu Met Met Tyr Ala
   275 280 285
   Thr Ile Trp Leu Arg Glu His Asn Arg Val Cys Asp Ile Leu Lys Gln
   290 295 300
   Glu His Pro Glu Trp Gly Asp Glu Gln Leu Phe Gln Thr Ser Arg Leu
   305 310 315 320
   Ile Leu Ile Gly Glu Thr Ile Lys Ile Val Ile Glu Asp Tyr Val Gln
   325 330 335
   His Leu Ser Gly Tyr His Phe Lys Leu Lys Phe Asp Pro Glu Leu Leu
   340 345 350
   Phe Asn Gln Gln Phe Gln Tyr Gln Asn Arg Ile Ala Ser Glu Phe Asn
   355 360 365
   Thr Leu Tyr His Trp His Pro Leu Leu Pro Asp Thr Phe Asn Ile Glu
   370 375 380
   Asp Gln Glu Tyr Ser Phe Lys Gln Phe Leu Tyr Asn Asn Ser Ile Leu
   385 390 395 400
   Leu Glu His Gly Leu Thr Gln Phe Val Glu Ser Phe Thr Arg Gln Ile
   405 410 415
   Ala Gly Arg Val Ala Gly Gly Arg Asn Val Pro Ile Ala Val Gln Ala
   420 425 430
   Val Ala Lys Ala Ser Ile Asp Gln Ser Arg Glu Met Lys Tyr Gln Ser
   435 440 445
   Leu Asn Glu Tyr Arg Lys Arg Phe Ser Leu Lys Pro Tyr Thr Ser Phe
   450 455 460
   Glu Glu Leu Thr Gly Glu Lys Glu Met Ala Ala Glu Leu Lys Ala Leu
   465                 470                 475                 480
   Tyr Ser Asp Ile Asp Val Met Glu Leu Tyr Pro Ala Leu Leu Val Glu
                   485                 490                 495
   Lys Pro Arg Pro Asp Ala Ile Phe Gly Glu Thr Met Val Glu Leu Gly
               500                 505                 510
   Ala Pro Phe Ser Leu Lys Gly Leu Met Gly Asn Pro Ile Cys Ser Pro
           515                 520                 525
   Gln Tyr Trp Lys Pro Ser Thr Phe Gly Gly Glu Val Gly Phe Lys Ile
       530                 535                 540
   Ile Asn Thr Ala Ser Ile Gln Ser Leu Ile Cys Asn Asn Val Lys Gly
   545                 550                 555                 560
   Cys Pro Phe Thr Ser Phe Asn Val Gln Asp Pro Gln Pro Thr Lys Thr
                   565                 570                 575
   Ala Thr Ile Asn Ala Ser Ala Ser His Ser Arg Leu Asp Asp Ile Asn
               580                 585                 590
   Pro Thr Val Leu Ile Lys Arg Arg Ser Thr Glu Leu
           595                 600

### (2) INFORMATION FOR SEQ ID NO:3:

(i) SEQUENCE CHARACTERISTICS:
   (A) LENGTH: 1834 base pairs
   (B) TYPE: nucleic acid
   (C) STRANDEDNESS: single
   (D) TOPOLOGY: linear
(ii) MOLECULE TYPE: cDNA
(vi) ORIGINAL SOURCE:
   (A) ORGANISM: Human PGHS-2
(xi) SEQUENCE DESCRIPTION: SEQ ID NO:3:
   CCGCTGCGAT GCTCGCCCGC GCCCTGCTGC TGTGCGCGGT CCTGGCGCTC AGCCATACAG     60
   CAAATCCTTG CTGTTCCCAC CCATGTCAAA ACCCAGGTGT ATGTATGAGT GTGGGATTTG    120
   ACCAGTATAA GTGCGATTGT ACCCGGACAG GATTCTATGG AGAAAACTGC TCAACACCGG    180
   AATTTTTGAC AAGAATAAAA TTATTTCTGA AACCCACTCC AAACACAGTG CACTACATAC    240
   TTACCCACTT CAAGGGATTT TGGAACGTTG TGAATAACAT TCCCTTCCTT CGAAATGCAA    300
   TTATGAGTTA TGTGTTGACA TCCAGATCAC ATTTGATTGA CAGTCCACCA ACTTACAATG    360
   CTGACTATGG CTACAAAAGC TGGGAAGCCT TCTCCAACCT CTCCTATTAT ACTAGAGCCC    420
   TTCCTCCTGT GCCTGATGAT TGCCCGACTC CCTTGGGTGT CAAAGGTAAA AAGCAGCTTC    480
   CTGATTCAAA TGAGATTGTG GAAAAATTGC TTCTAAGAAG AAAGTTCATC CCTGATCCCC    540
   AGGGCTCAAA CATGATGTTT GCATTCTTTG CCCAGCACTT CACGCATCAG TTTTTCAAGA    600
   CAGATCATAA GCGAGGGCCA GCTTTCACCA ACGGGCTGGG CCATGGGGTG GACTTAAATC    660
   ATATTTACGG TGAAACTCTG GCTAGACAGC GTAAACTGCG CCTTTTCAAG GATGGAAAAA    720
   TGAAATATCA GATAATTGAT GGAGAGATGT ATCCTCCCAC AGTCAAAGAT ACTCAGGCAG    780
   AGATGATCTA CCCTCCTCAA GTCCCTGAGC ATCTACGGTT TGCTGTGGGG CAGGAGGTCT    840
   TTGGTCTGGT GCCTGGTCTG ATGATGTATG CCACAATCTG GCTGCGGGAA CACAACAGAG    900
   TATGCGATGT GCTTAAACAG GAGCATCCTG AATGGGGTGA TGAGCAGTTG TTCCAGACAA    960
   GCAGGCTAAT ACTGATAGGA GAGACTATTA AGATTGTGAT TGAAGATTAT GTGCAACACT   1020
   TGAGTGGCTA TCACTTCAAA CTGAAGTTTG ACCCAGAACT ACTTTTCAAC AAACAGTTCC   1080
   AGTACCAAAA TCGTATTGCT GCTGAATTTA ACACCCTCTA TCACTGGCAT CCCCTTCTGC    1140
   CTGACACCTT TCAAATTCAT GACCAGAAAT ACAACTATCA ACAGTTTATC TACAACAACT    1200
   CTATATTGCT GGAACATGGA ATTACCCAGT TTGTTGAATC ATTCACCAGG CAGATTGCTG    1260
   GCAGGGTTGC TGGTGGTAGG AATGTTCCAC CCGCAGTACA GAAAGTATCA CAGGCTTCCA    1320
   TTGACCAGAG CAGGCAGATG AAATACCAGT CTTTTAATGA GTACCGCAAA CGCTTTATGC    1380
   TGAAGCCCTA TGAATCATTT GAAGAACTTA CAGGAGAAAA GGAAATGTCT GCAGAGTTGG    1440
   AAGCACTCTA TGGTGACATC GATGCTGTGG AGCTGTATCC TGCCCTTCTG GTAGAAAAGC    1500
   CTCGGCCAGA TGCCATCTTT CCTCAAACCA TCCTACAACT TGGAGCACCA TTCTCCTTGA    1560
   AACCACTTAT GGGTAATGTT ATATGTTCTC CTGCCTACTG GAAGCCAAGC ACTTTTGGTG    1620
   GAGAAGTGGG TTTTCAAATC ATCAACACTG CCTCAATTCA GTCTCTCATC TGCAATAACG    1680
   TGAAGGGCTG TCCCTTTACT TCATTCAGTG TTCCAGATCC AGAGCTCATT AAAACAGTCA    1740
   CCATCAATGC AAGTTCTTCC CGCTCCGGAC TAGATGATAT CAATCCCACA CTACTACTAA    1800
   AAGAACGTTC GACTGAACTG TAGAAGTCTA ATAC                                1834

### (2) INFORMATION FOR SEQ ID NO:4:

(i) SEQUENCE CHARACTERISTICS:
   (A) LENGTH: 604 amino acids
   (B) TYPE: amino acid
   (D) TOPOLOGY: linear
(ii) MOLECULE TYPE: protein
(vi) ORIGINAL SOURCE:
   (A) ORGANISM: Amino acid sequence for Human PGHS-2
(xi) SEQUENCE DESCRIPTION: SEQ ID NO:4:
   Met Leu Ala Arg Ala Leu Leu Leu Cys Ala Val Leu Ala Leu Ser His
   1               5                   10                  15
   Thr Ala Asn Pro Cys Cys Ser His Pro Cys Gln Asn Arg Gly Val Cys
               20                  25                  30
   Met Ser Val Gly Phe Asp Gln Tyr Lys Cys Asp Cys Thr Arg Thr Gly
           35                  40                  45
   Phe Tyr Gly Glu Asn Cys Ser Thr Pro Glu Phe Leu Thr Arg Ile Lys
       50                  55                  60
   Leu Phe Leu Lys Pro Thr Pro Asn Thr Val His Tyr Ile Leu Thr His
   65                  70                  75                  80
   Phe Lys Gly Phe Trp Asn Val Val Asn Asn Ile Pro Phe Leu Arg Asn
                   85                  90                  95
   Ala Ile Met Ser Tyr Val Leu Thr Ser Arg Ser His Leu Ile Asp Ser
               100                 105                 110
   Pro Pro Thr Tyr Asn Ala Asp Tyr Gly Tyr Lys Ser Trp Glu Ala Phe
           115                 120                 125
   Ser Asn Leu Ser Tyr Tyr Thr Arg Ala Leu Pro Pro Val Pro Asp Asp
       130                 135                 140
   Cys Pro Thr Pro Leu Gly Val Lys Gly Lys Lys Gln Leu Pro Asp Ser
   145                 150                 155                 160
   Asn Glu Ile Val Glu Lys Leu Leu Leu Arg Arg Lys Phe Ile Pro Asp
                   165                 170                 175
   Pro Gln Gly Ser Asn Met Met Phe Ala Phe Phe Ala Gln His Phe Thr
               180                 185                 190
   His Gln Phe Phe Lys Thr Asp His Lys Arg Gly Pro Ala Phe Thr Asn
           195                 200                 205
   Gly Leu Gly His Gly Val Asp Leu Asn His Ile Tyr Gly Glu Thr Leu
       210                 215                 220
   Ala Arg Gln Arg Lys Leu Arg Leu Phe Lys Asp Gly Lys Met Lys Tyr
   225                 230                 235                 240
   Gln Ile Ile Asp Gly Glu Met Tyr Pro Pro Thr Val Lys Asp Thr Gln
                   245                 250                 255
   Ala Glu Met Ile Tyr Pro Pro Gln Val Pro Glu His Leu Arg Phe Ala
               260                 265                 270
   Val Gly Gln Glu Val Phe Gly Leu Val Pro Gly Leu Met Met Tyr Ala
           275                 280                 285
   Thr Ile Trp Leu Arg Glu His Asn Arg Val Cys Asp Val Leu Lys Gln
       290                 295                 300
   Glu His Pro Glu Trp Gly Asp Glu Gln Leu Phe Gln Thr Ser Arg Leu
   305                 310                 315                 320
   Ile Leu Ile Gly Glu Thr Ile Lys Ile Val Ile Glu Asp Tyr Val Gln
                   325                 330                 335
   His Leu Ser Gly Tyr His Phe Lys Leu Lys Phe Asp Pro Glu Leu Leu
               340                 345                 350
   Phe Asn Lys Gln Phe Gln Tyr Gln Asn Arg Ile Ala Ala Glu Phe Asn
           355                 360                 365
   Thr Leu Tyr His Trp His Pro Leu Leu Pro Asp Thr Phe Gln Ile His
       370                 375                 380
   Asp Gln Lys Tyr Asn Tyr Gln Gln Phe Ile Tyr Asn Asn Ser Ile Leu
   385                 390                 395                 400
   Leu Glu His Gly Ile Thr Gln Phe Val Glu Ser Phe Thr Arg Gln Ile
                   405                 410                 415
   Ala Gly Arg Val Ala Gly Gly Arg Asn Val Pro Pro Ala Val Gln Lys
               420                 425                 430
   Val Ser Gln Ala Ser Ile Asp Gln Ser Arg Gln Met Lys Tyr Gln Ser
           435                 440                 445
   Phe Asn Glu Tyr Arg Lys Arg Phe Met Leu Lys Pro Tyr Glu Ser Phe
       450                 455                 460
   Glu Glu Leu Thr Gly Glu Lys Glu Met Ser Ala Glu Leu Glu Ala Leu
   465                 470                 475                 480
   Tyr Gly Asp Ile Asp Ala Val Glu Leu Tyr Pro Ala Leu Leu Val Glu
                   485                 490                 495
   Lys Pro Arg Pro Asp Ala Ile Phe Gly Glu Thr Met Val Glu Val Gly
               500                 505                 510
   Ala Pro Phe Ser Leu Lys Gly Leu Met Gly Asn Val Ile Cys Ser Pro
           515                 520                 525
   Ala Tyr Trp Lys Pro Ser Thr Phe Gly Gly Glu Val Gly Phe Gln Ile
       530                 535                 540
   Ile Asn Thr Ala Ser Ile Gln Ser Leu Ile Cys Asn Asn Val Lys Gly
   545                 550                 555                 560
   Cys Pro Phe Thr Ser Phe Ser Val Pro Asp Pro Glu Leu Ile Lys Thr
                   565                 570                 575
   Val Thr Ile Asn Ala Ser Ser Ser Arg Ser Gly Leu Asp Asp Ile Asn
               580                 585                 590
   Pro Thr Val Leu Leu Lys Glu Arg Ser Thr Glu Leu
           595                 600

### (2) INFORMATION FOR SEQ ID NO:5:

(i) SEQUENCE CHARACTERISTICS:
   (A) LENGTH: 604 amino acids
   (B) TYPE: amino acid
   (C) STRANDEDNESS: single
   (D) TOPOLOGY: linear
(ii) MOLECULE TYPE: protein
(vi) ORIGINAL SOURCE:
   (A) ORGANISM: Amino acid sequence Human PGHS-2
(xi) SEQUENCE DESCRIPTION: SEQ ID NO:5:

### (2) INFORMATION FOR SEQ ID NO:6:

(i) SEQUENCE CHARACTERISTICS:
   (A) LENGTH: 1819 base pairs
   (B) TYPE: nucleic acid
   (C) STRANDEDNESS: single
   (D) TOPOLOGY: linear
(ii) MOLECULE TYPE: cDNA
(vi) ORIGINAL SOURCE:
   (A) ORGANISM: Human PGHS-1 Gene
(ix) FEATURE:
   (A) NAME/KEY: CDS
   (B) LOCATION: 8..1804
(xi) SEQUENCE DESCRIPTION: SEQ ID NO:6:
   CCGCGCC ATG AGC CGG AGT CTC TTG CTC CGG TTC TTG CTG TTG CTG CTC        49
   CTG CTC CCG CCG CTC CCC GTC CTG CTC GCG GAC CCA GGG GCG CCC ACG        97
   CCA GTG AAT CCC TGT TGT TAC TAT CCA TGC CAG CAC CAG GGC ATC TGT       145
   GTC CGC TTC GGC CTT GAC CGC TAC CAG TGT GAC TGC ACC CGC ACG GGC       193
   TAT TCC GGC CCC AAC TGC ACC ATC CCT GGC CTG TGG ACC TGG CTC CGG       241
   AAT TCA CTG CGG CCC AGC CCC TCT TTC ACC CAC TTC CTG CTC ACT CAC       289
   GGG CGC TGG TTC TGG GAG TTT GTC AAT GCC ACC TTC ATC CGA GAG ATG       337
   CTC ATG CTC CTG GTA CTC ACA GTG CGC TCC AAC CTT ATC CCC AGT CCC       385
   CCC ACC TAC AAC TCT GCA CAT GAC TAC ATC AGC TGG GAG TCT TTC TCC      433
   AAC GTG AGC TAT TAC ACT CGT ATT CTG CCC TCT GTG CCT AAA GAT TGC      481
   CCC ACA CCC ATG GGA ACC AAA GGG AAG AAG CAG TTG CCA GAT GCC CAG      529
   CTC CTG GCC CGC CGC TTC CTG CTC AGG AGG AAG TTC ATA CCT GAC CCC      577
   CAA GGC ACC AAC CTC ATG TTT GCC TTC TTT GCA CAA CAC TTC ACC CAC      625
   CAG TTC TTC AAA ACT TCT GGC AAG ATG GGT CCT GGC TTC ACC AAG GCC      673
   TTG GGC CAT GGG GTA GAC CTC GGC CAC ATT TAT GGA GAC AAT CTG GAG      721
   CGT CAG TAT CAA CTG CGG CTC TTT AAG GAT GGG AAA CTC AAG TAC CAG      769
   GTG CTG GAT GGA GAA ATG TAC CCG CCC TCG GTA GAA GAG GCG CCT GTG      817
   TTG ATG CAC TAC CCC CGA GGC ATC CCG CCC CAG AGC CAG ATG GCT GTG      865
   GGC CAG GAG GTG TTT GGG CTG CTT CCT GGG CTC ATG CTG TAT GCC ACG      913
   CTC TGG CTA CGT GAG CAC AAC CGT GTG TGT GAC CTG CTG AAG GCT GAG      961
   CAC CCC ACC TGG GGC GAT GAG CAG CTT TTC CAG ACG ACC CGC CTC ATC     1009
   CTC ATA GGG GAG ACC ATC AAG ATT GTC ATC GAG GAG TAC GTG CAG CAG      1057
   CTG AGT GGC TAT TTC CTG CAG CTG AAA TTT GAC CCA GAG CTG CTG TTC      1105
   GGT GTC CAG TTC CAA TAC CGC AAC CGC ATT GCC ACG GAG TTC AAC CAT      1153
   CTC TAC CAC TGG CAC CCC CTC ATG CCT GAC TCC TTC AAG GTG GGC TCC      1201
   CAG GAG TAC AGC TAC GAG CAG TTC TTG TTC AAC ACC TCC ATG TTG GTG      1249
   GAC TAT GGG GTT GAG GCC CTG GTG GAT GCC TTC TCT CGC CAG ATT GCT      1297
   GGC CGG ATC GGT GGG GGC AGG AAC ATG GAC CAC CAC ATC CTG CAT GTG      1345
   GCT GTG GAT GTC ATC AGG GAG TCT CGG GAG ATG CGG CTG CAG CCC TTC      1393
   AAT GAG TAC CGC AAG AGG TTT GGC ATG AAA CCC TAC ACC TCC TTC CAG      1441
   GAG CTC GTA GGA GAG AAG GAG ATG GCA GCA GAG TTG GAG GAA TTG TAT      1489
   GGA GAC ATT GAT GCG TTG GAG TTC TAC CCT GGA CTG CTT CTT GAA AAG      1537
   TGC CAT CCA AAC TCT ATC TTT GGG GAG AGT ATG ATA GAG ATT GGG GCT      1585
   CCC TTT TCC CTC AAG GGT CTC CTA GGG AAT CCC ATC TGT TCT CCG GAG      1633
   TAC TGG AAG CCG AGC ACA TTT GGC GGC GAG GTG GGC TTT AAC ATT GTC       1681
   AAG ACG GCC ACA CTG AAG AAG CTG GTC TGC CTC AAC ACC AAG ACC TGT       1729
   CCC TAC GTT TCC TTC CGT GTG CCG GAT GCC AGT CAG GAT GAT GGG CCT       1777
   GCT GTG GAG CGA CCA TCC ACA GAG CTC TGAGGGGCAG GAAAG                  1819

### (2) INFORMATION FOR SEQ ID NO:7:

(i) SEQUENCE CHARACTERISTICS:
   (A) LENGTH: 10 amino acids
   (B) TYPE: amino acid
   (D) TOPOLOGY: linear
(ii) MOLECULE TYPE: peptide
(xi) SEQUENCE DESCRIPTION: SEQ ID NO:7:
   Thr Ile Trp Leu Arg Glu His Asn Arg Val
   1               5                   10

### (2) INFORMATION FOR SEQ ID NO:8:

(i) SEQUENCE CHARACTERISTICS:
   (A) LENGTH: 5 amino acids
   (B) TYPE: amino acid
   (D) TOPOLOGY: linear
(ii) MOLECULE TYPE: peptide
(xi) SEQUENCE DESCRIPTION: SEQ ID NO:8:
   Lys Ala Leu Gly His
   1               5

### (2) INFORMATION FOR SEQ ID NO:9:

(i) SEQUENCE CHARACTERISTICS:
   (A) LENGTH: 5 amino acids
   (B) TYPE: amino acid
   (D) TOPOLOGY: linear
(ii) MOLECULE TYPE: peptide
(xi) SEQUENCE DESCRIPTION: SEQ ID NO:9:
   Arg Gly Leu Gly His
   1               5

### (2) INFORMATION FOR SEQ ID NO:10:

(i) SEQUENCE CHARACTERISTICS:
   (A) LENGTH: 28 base pairs
   (B) TYPE: nucleic acid
   (C) STRANDEDNESS: single
   (D) TOPOLOGY: linear
(ii) MOLECULE TYPE: cDNA
(vi) ORIGINAL SOURCE:
   (A) ORGANISM: Human PGHS-1 PCR Primer
(xi) SEQUENCE DESCRIPTION: SEQ ID NO:10:
   CTTACCCGAA GCTTGCGCCA TGAGCCGG                    28

### (2) INFORMATION FOR SEQ ID NO:11:

(i) SEQUENCE CHARACTERISTICS:
   (A) LENGTH: 28 base pairs
   (B) TYPE: nucleic acid
   (C) STRANDEDNESS: single
   (D) TOPOLOGY: linear
(ii) MOLECULE TYPE: cDNA
(vi) ORIGINAL SOURCE:
   (A) ORGANISM: Human PGHS-1 PCR primer
(xi) SEQUENCE DESCRIPTION: SEQ ID NO:11:
   TTCGTTAGCG GCCGCTGCCC CTCAGAGC 28

### (2) INFORMATION FOR SEQ ID NO:12:

(i) SEQUENCE CHARACTERISTICS:
   (A) LENGTH: 29 base pairs
   (B) TYPE: nucleic acid
   (C) STRANDEDNESS: single
   (D) TOPOLOGY: linear
(ii) MOLECULE TYPE: cDNA
(vi) ORIGINAL SOURCE:
   (A) ORGANISM: Human PGHS-2 PCR Primers
(xi) SEQUENCE DESCRIPTION: SEQ ID NO:12:
   TCATTCTAAG CTTCCGCTGC GATGCTCGC 29

### (2) INFORMATION FOR SEQ ID NO:13:

(i) SEQUENCE CHARACTERISTICS:
   (A) LENGTH: 29 base pairs
   (B) TYPE: nucleic acid
   (C) STRANDEDNESS: single
   (D) TOPOLOGY: linear
(ii) MOLECULE TYPE: cDNA
(vi) ORIGINAL SOURCE:
   (A) ORGANISM: Human PGHS-2 PCR primers
(xi) SEQUENCE DESCRIPTION: SEQ ID NO:13:
   GATCATGCGG CCGCATTAGA CTTCTACAG 29

## Description

### Cross-Reference to Related Applications

This application is a continuation-in-part of U.S. patent application Serial No. 7/983,835, filed December 1, 1992 which in turn is a continuation-in-part of U.S. patent application Serial No. 7/949,780 filed September 22, 1992.

### Background of the Invention

This invention was made with government support under grant number DK 16177, awarded by the National Institutes of Health. The government has certain rights in the invention.

Prostaglandins (which include PGE₂, PGD₂, PGF_{2α}, PGI₂ and other related compounds) represent a diverse group of autocrine and paracrine hormones that are derived from the metabolism of fatty acids. They belong to a family of naturally occurring eicosanoids (prostaglandins, thromboxanes and leukotrienes) which are not stored as such in cells, but are biosynthesized on demand from arachidonic acid, a 20-carbon fatty acid that is derived from the breakdown of cell-membrane phospholipids. Under normal circumstances, the eicosanoids are produced at low levels to serve as important mediators of many and diverse cellular functions which can be very different in different types of cells. However, the prostaglandins also play critical roles in pathophysiology. In particular, inflammation is both initiated and maintained, at least in part, by the overproduction of prostaglandins in injured cells. The central role that prostaglandins play in inflammation is underscored by the fact that those aspirin-like non-steroidal anti-inflammatory drugs (NSAIDS) that are most effective in the therapy of many pathological inflammatory states all act by inhibiting prostaglandin synthesis. Unfortunately, the use of these drugs is often limited by the side effects (gastrointestinal bleeding, ulcers, renal failure, and others) that result from the undesirable reduction in prostaglandins in normal cells that now suffer from a lack of those autocrine and paracrine functions that are required for the maintenance of normal physiology. The development of new agents that will act more specifically by achieving a reduction in prostaglandins in inflamed cells without altering prostaglandin production in other cells is one of the major goals for future medicinal therapy.

The cyclooxygenase reaction is the first step in the prostaglandin synthetic pathway; an enzyme (PGHS) with prostaglandin G/H synthetic activity converts arachidonic acid into the endoperoxide PGG₂, which then breaks down to PGH₂ (the two reactions are carried out by a single enzyme). PGH₂ is in turn metabolized by one or more prostaglandin synthases (PGE₂ synthase, PGD₂ synthase, etc.) to generate the final "2-series" prostaglandins, PGE₂, PGD₂, PGF_{2α}, PGI₂ and others which include the thromboxanes, TXA₂. The first step (PGHS) is the one that is rate-limiting for prostaglandin synthesis. As such, the PGHS-mediated reaction is the principal target for anti-inflammatory drug action; and it is inhibition of PGHS activity that accounts for the activity of the NSAIDS (aspirin, indomethacin, naproxen and others that a) limit the overproduction of prostaglandins in inflammation (the desired therapeutic goal) and b) reduce the normal production of prostaglandins in uninflamed cells (which produces the undesirable side effects).

In addition to the abnormal changes associated with inflammation, multiple other factors are known to influence prostaglandin production under experimental conditions. These include growth factors, cAMP, tumor promoters, src activation and interleukins 1 and 2, all of which increase overall cellular PGHS activity. The adrenal glucocorticoid hormones and related synthetic anti-inflammatory steroids also inhibit prostaglandin synthesis, but their metabolic site of action is not well defined.

Human, ovine, and murine cDNAs have been cloned for PGHS-1. All show similar sequences and hybridize with 2.8-3.0-kb mRNAs on Northern blots. However, several research groups have recently identified and predicted the sequence of a protein reported to be related to PGHS-1 in some manner. In 1990, J.S. Han et al., in PNAS USA, 87, 3373 (May 1990), reported changes in protein synthesis caused by the polypeptide pp6O^{v-src}, following infection of BALB/c 3T3 fibroblasts by Rous sarcoma virus temperature-sensitive mutant strain LA90. Giant two-dimensional gel electrophoresis detected induction of a 72-74 kDa protein doublet that is recognized by anticyclooxygenase antibodies. Synthesis of this doublet was also transiently increased by exposure to platelet-derived growth factor and inhibited by dexamethasone treatment. These changes in protein synthesis were strongly correlated with changes in cyclooxygenase activity. The protein doublet was also seen in mouse C127 fibroblasts where its synthesis was found to be regulated by serum and dexamethasone and correlated with cyclooxygenase activity. See, M.K. O'Banion et al., J. Biol. Chem., 266, 23261 (Dec. 5, 1991).

W. Xie et al., in PNAS USA, 88, 2692 (April 1991) followed their earlier report of the isolation of a set of cDNAs corresponding to pp60^{v-src} - inducible immediate - early genes in chicken embryo fibroblasts, with a report that one of the genes, designated CEF-147, encodes a protein related to PGHS-1. They termed the pp60^{v-src} - inducible form "miPGHS_{ch}", for mitogen-inducible PGHS_{chicken}. Although Xie et al. speculated that prostaglandin synthesis may play a role in src product-mediated cellular transformation, their experiments did not permit them to discriminate between miPGHS_{ch} as a second cyclooxygenase or simply as the chicken homolog of sheep PGHS-1, "PGHSₒᵥ".

In a separate set of experiments, D.A. Kujubu et al., in J. Biol. Chem., 266, 12866 (1991) reported that one of the primary response genes cloned from mitogen-responding Swiss 3T3 cells (TIS1O) has a long 3'-untranslated region and encodes a "predicted" 66 kDa protein which is about 60% identical to mouse PGHS-1. The sequence of this putative protein was essentially identical to that derived by Xie et al. On the basis of sequence similarities, Kujubu et al. speculated that the enzymatic activity of the protein encoded by the TIS1O gene would be likely to be "similar" to enzymatic activity of other types of mammalian PGHS-1. They concluded that "[p]roof of this conjecture, however, awaits the heterologous expression of this gene production from an expressible plasmid and the direct measurement of cyclooxygenase activity in transfected cells and/or purified preparations of the TIS1O protein."

There is increasing emphasis on the development of methods for the modulation and evaluation of the activity of the prostaglandin synthetic pathway. As noted above, nonsteroidal anti-inflammatory agents, such as aspirin and indomethacin, inhibit the cyclooxygenase which converts arachidonic acid into PGG₂ and PGH₂. Therefore, there is a need for improved methods to study the effectiveness of existing anti-inflammatory drugs and to evaluate the effectiveness of potential anti-inflammatory agents, at the molecular level, as well as for reagents for use in such methods.

### Summary of the Invention

The present invention provides a mammalian cell line which contains a chromosomally integrated, recombinant DNA sequence, which DNA sequence expresses human, glucocorticoid-regulated inflammatory PGHS corresponding to SEQ ID NO. 4, and which cell line does not significantly express autologous PGHS-1 or PGHS-2 activity. For brevity, glucocorticoid-regulated inflammatory PGHS will hereinafter be referred to as "griPGHS" or "PGHS-2", and the art-recognized mammalian PGHS encoded by the 2.8-3.0 kb mRNA (EC 1.14.99.1) will be referred to as "constitutive cyclooxygenase," or "constitutive PGHS," or "PGHS-1." The recitation that there is no "autologous PGHS-1 or PGHS-2 activity" relates to the inability of the cell line to express PGHS activity apart from that expressed by the recombinant DNA sequence. Autologous PGHS activity may also be referred to as "endogenous" PGHS activity in the art.

This invention is a result of our discovery that the 72-74 kDA cyclooxygenase reported by Han et al., the miPGHS_{ch} reported by Xie et al., and the TIS10 protein reported by Kujubu et al. are essentially identical and represent a second cyclooxygenase, which second form is the primary target for inhibition by glucocorticoids and is also a target for inhibition by non-steroidal anti-inflammatory agents.

In December of 1991, we reported the synthesis of a 70 kilodalton (kDa) protein in C127 mouse fibroblasts, via a mouse 4 kilobase (Kb) mRNA, and also published the derived amino acid sequence. The protein encoded by the 4-kb mRNA shows 80% amino acid identify with the previously known mouse PGHS-1 protein product in a sequenced 240 base region. See, M. Kerry O'Banion et al., J. Biol. Chem., 35, 23261 (December 5, 1991).

The 70 kDa protein, designated griPGHS or PGHS-2 herein, was determined to be a discrete form of cyclooxygenase by several assays. The protein was precipitated by anti-PGHS serum, its synthesis and concomitant cyclooxygenase levels are rapidly induced by serum, and the induction is inhibited by dexamethasone. The regulation of PGHS-2 synthesis was found not to arise from alterations in the level of the 2.8-kb PGHS-1 mRNA, but resulted from changes in the level of a 4-kb mRNA species. This latter species is barely detectable with a 2.8-kb PGHS-1 DNA probes in cells treated with serum, but accumulates to significant levels in cells treated with cycloheximide or calcium ionophore. In contrast, there was no change in the level of the 2.8-kb mRNA which encodes PGHS-1 or "constitutive PGHS" as observed following treatment with serum, dexamethasone or cycloheximide. Finally, by hybridization analysis, we proved that the 4-Kb mRNA represented the product of a gene that is distinct from the gene giving rise to the 2.8-Kb mRNA.

These observations indicated that there are two cyclooxygenase genes; one constitutively expressed as a 2.8-kb mRNA, and a second giving rise to a growth factor- and glucocorticoid-regulated 4-kb mRNA which encodes PGHS-2. It is believed that expression of the latter 4-kb RNA and concomitantly increased PGHS-2 levels are primarily, if not entirely, responsible for the enhanced prostaglandin synthesis that is responsible, directly or indirectly, for many of the adverse effects of inflammation.

The present PGHS-2-synthesizing transgenic cell line is useful for evaluating the action of a potential bioactive agent on the inflammatory cyclooxygenase, since the elevated levels of prostaglandins that are a primary hallmark of inflammation and account for much of the adverse effects of inflammation, result from increases in the level of PGHS-2, rather than in changes in constitutively expressed cyclooxygenase, PGHS-1.

The present invention also utilises a second transgenic mammalian cell line which contains a chromosomally integrated, recombinant DNA sequence, wherein said DNA sequence expresses mammalian, preferably human, PGHS-1, and wherein said DNA sequence does not express any PGHS-2, and wherein said cell line also preferably does not express autologous PGHS-1 or PGHS-2 activity. This second cell line is also preferably a primate, murine or human cell line.

Thus, the present invention also provides a method to evaluate the relative inhibitory activity of a compound to selectively inhibit PGHS-2 versus PGHS-1, and thus to specifically inhibit the elevated prostaglandin synthesis that occurs in inflamed mammalian tissues, preferably human tissues, or in other physiological or pathological conditions in a mammalian host, preferably a human host, in which the PGHS-2 is elevated and the constitutive PGHS-1 is not. This assay comprises contacting the present PGHS-2- expressing transgenic cell line or a microsomal extract thereof with a preselected amount of the compound in a suitable culture medium or buffer, adding arachidonic acid to the mixture, and measuring the level of synthesis of a PGHS-mediated arachidonic acid metabolite, i.e., thromboxane synthesis, prostaglandin synthesis, e.g., the synthesis of PGE₂ or the synthesis of any other metabolite unique to the cyclooxygenase pathway, by said cell line, or said microsomal extract, as compared to a control cell line or portion of microsomal extract in the absence of said compound. The compound can be evaluated for its ability to selectively inhibit PGHS-1 or PGHS-2 by performing a second assay employing the above-described steps, but substituting the PGHS-1-expressing transgenic cell line for the PGHS-2- expressing cell line of the invention.

More specifically, the present invention provides a method of determining the ability of a compound to inhibit prostaglandin synthesis catalyzed by PGHS-2 or PGHS-1 in mammalian cells comprising:
(a) adding a first preselected amount of said compound to a first transgenic mammalian cell line in culture medium, which cell line contains a chromosomally integrated, recombinant DNA sequence, wherein said DNA sequence expresses human PGHS-2 having an aminoacid sequence corresponding to SEQ ID NO. 4, and wherein said DNA sequence does not express PGHS-1, and wherein said cell line does not express autologous PGHS-1 or PGHS-2 activity;
(b) adding arachidonic acid to said culture medium;
(c) measuring the level of a PGHS-mediated arachidonic acid metabolite synthesized by said first cell line;
(d) comparing said level with the level of said metabolite synthesized by said first cell line in the absence of said compound;
(e) adding a second preselected amount of said compound to a second transgenic mammalian cell line in culture medium, which cell line contains chromosomally integrated, recombinant DNA sequence, wherein said DNA sequence expresses mammalian PGHS-1, and wherein said DNA sequence does not express any PGHS-2, and wherein said cell line does not express autologous PGHS-1 or PGHS-2 activity;
(f) adding arachidonic acid to said culture medium of step (e);
(g) measuring the level of a PGHS-mediated arachidonic acid metabolite synthesized by said second cell line; and
(h) comparing said level with the level of said metabolite synthesized by said second cell line in the absence of said compound.

Of course, a comparison of the relative ability of the compound to inhibit metabolite, i.e., prostaglandin, synthesis as determined in steps (d) and (h), provides a direct measure of the selectivity of the compound with respect to the inhibition of PGHS-2 and PGHS-1, respectively.

Thus, it can be seen that since PGHS-2 levels are increased in cell models of inflammation, and since reductions in PGHS-1 are believed to cause the undesirable side effects of those drugs which inhibit cyclooxygenase activity, it will be necessary to evaluate the actions of individual drugs on both PGHS-2 and PGHS-1 using the claimed methods. Previous estimates of the anti-inflammatory actions of drug candidates based on previous *in vitro* assays might be misleading, since activities of the constitutive versus the inflammatory cyclooxygenase were not distinguished. Using the stable cell lines of the invention, which express either the constitutive cyclooxygenase encoded by the 2.8-kb mRNA or the inducible cyclooxygenase encoded by the 4-Kb mRNA which upon translation provides a protein with an amino acid sequence corresponding to SEQ ID NO. 4, and analyzing dose response curves performed on each cell line will allow a drug's specificity for PGHS-1 or PGHS-2 to be determined. Studies comparing drug actions against the PGHS-1 or PGHS-2 may shed light on the unique clinical uses of the various nonsteroidal anti-inflammatory agents. They may also allow for titration of drug doses to inhibit PGHS-2 activity and leave other cyclooxygenase activity intact. Finally, the availability of the cell lines of the invention provides a mechanism for the discovery and/or development of agents that are specific inhibitors of the PGHS-2. Such agents might be predicted to have the important anti-inflammatory actions of current drugs without the significant side effects that may result from a general inhibition of prostaglandin biosynthesis.

The present invention also comprises an isolated DNA sequence (gene) encoding biologically active human PGHS-2 and the isolated, essentially pure human PGHS-2 encoded thereby.

### Brief Description of the Figures

Figure 1 depicts the cDNA (SEQ ID NO:1) and predicted amino acid sequence (SEQ ID NO:2) of murine griPGHS ("PGHS-2"). Based on a transcription start site determined by primer extension at -24, the numbering of this sequence starts at 25. A predicted signal peptide cleavage site between amino acids 17 and 18 is marked with an arrowhead. The position of the putative aspirin-modified serine is indicated by a circle, and potential N-glycosylation sites are double underlined.

Figure 2 is a schematic depiction comparing the cDNA and protein sequences for the murine 2.8- and 4.1kb RNA-encoded cyclooxygenases.

Figure 3 is a photographic depiction of autoradiographies obtained by Northern blotting monitoring the expression of the genes encoding griPGHS and the constitutive PGHS-1, as expressed in human monocytes, in response to interleukin-1 treatment, a known mediator of inflammation.

Figure 4 is a schematic depiction of griPGHS expression vector construction. The dots in the 3' untranslated region of griPGHS indicate the location of 5'-AUUUA-3' mRNA destabilizing sequences.

Figure 5 is a graphic depiction of the inhibition of murine griPGHS activity in stable transfected mammalian cell lines by preselected amounts of several non-steroidal anti-inflammatory drugs.

Figure 6 depicts the nucleotide sequence of the human PGHS-2 gene (SEQ ID NO:3).

Figure 7 depicts a comparison between the amino acid sequence of human PGHS-2 of the present invention (upper sequence) (SEQ ID NO: 4) and the amino acid sequence published by Hla et al. (lower sequence) (SEQ ID NO: 5). The sequences are given in standard single letter code.

Figure 8 is a graphical depiction of the inhibition of human PGHS-2 activity in stably transformed COS cells by four non-steroidal anti-inflammatory drugs (NSAID).

Figure 9 is a graphical depiction of the inhibition of human PGHS-1 activity in stably transformed COS cells by four NSAID.

### Detailed Description of the Invention

The present invention relates to a transgenic cell line containing a chromosomally integrated recombinant DNA sequence encoding the regulated inflammatory human PGHS-2 corresponding to SEQ ID NO. 4 which cell line further does not express autologous PGHS-1 or PGHS-2, apart from that encoded by the recombinant DNA sequence. The recombinant DNA also does not encode constitutive PGHS-1 (EC 1.14.99.1).

A preferred embodiment of the present invention is a transgenic mammalian cell line which contains a chromosomally integrated, genetically-engineered ("recombinant") DNA sequence, which DNA sequence expresses PGHS-2 corresponding to SEQ ID NO. 4, but does not express constitutive mammalian PGHS-1, and wherein said cell line also does not express autologous PGHS-1 or PGHS-2. The cell line is preferably of human primate origin, such as the exemplified monkey kidney COS cell line, but cell lines derived from other species may be employed, including chicken, hamster, murine, ovine and the like.

"Transgenic" is used herein to include any cell or cell line, the genotype of which has been altered by the presence of a recombinant DNA sequence, which DNA sequence has also been referred to in the art of genetic engineering as "heterologous DNA," "exogenous DNA," "genetically engineered" or "foreign DNA," wherein said DNA was introduced into the genotype or genome of the cell or cell line by a process of genetic engineering.

As used herein, the term "recombinant DNA sequence" refers to a DNA sequence that has been derived or isolated from any source, that may be subsequently chemically altered, and later introduced into mammalian cells. An example of a recombinant DNA sequence "derived" from a source, would be a DNA sequence that is identified as a useful fragment within a given organism, and which is then chemically synthesized in essentially pure form. An example of such DNA sequence "isolated" from a source would be a useful DNA sequence that is excised or removed from said source by chemical means, e.g., by the use of restriction endonucleases, so that it can be further manipulated, e.g., amplified, for use in the invention, by the methodology of genetic engineering.

Therefore, "recombinant DNA sequence" includes completely synthetic DNA, semi-synthetic DNA, DNA isolated from biological sources, and DNA derived from introduced RNA. Generally, the recombinant DNA sequence is not originally resident in the genotype which is the recipient of the DNA sequence, or it is resident in the genotype but is not expressed.

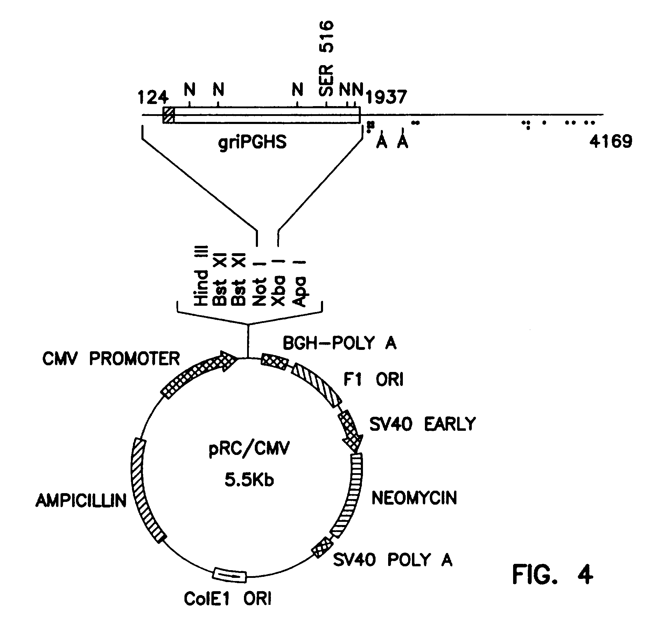

The isolated recombinant DNA sequence used for transformation herein may be circular or linear, doublestranded or single-stranded. Generally, the DNA sequence is chimeric linear DNA, or is in a plasmid or viral expression vector, that can also contain coding regions flanked by regulatory sequences which promote the expression of the recombinant DNA present in the resultant cell line. For example, the recombinant DNA sequence may itself comprise or consist of a promoter that is active in mammalian cells, or may utilize a promoter already present in the genotype that is the transformation target. Such promoters include the CMV promoter depicted in Figure 4, as well as the SV 40 late promoter and retroviral LTRs (long terminal repeat elements).

The general methods for constructing recombinant DNA which can transform target cells are well known to those skilled in the art, and the same compositions and methods of construction may be utilized to produce the DNA useful herein. For example, J. Sambrook et al., Molecular Cloning: A Laboratory Manual, Cold Spring Harbor Laboratory Press (2d ed., 1989), provides suitable methods of construction.

Aside from recombinant DNA sequences that serve as transcription units for PGHS-1, PGHS-2 or other portions thereof, a portion of the recombinant DNA may be untranscribed, serving a regulatory or a structural function.

The recombinant DNA sequence to be introduced into the cells further will generally contain either a selectable marker gene or a reporter gene or both to facilitate identification and selection of transformed cells. Alternatively, the selectable marker may be carried on a separate piece of DNA and used in a co-transformation procedure. Both selectable markers and reporter genes may be flanked with appropriate regulatory sequences to enable expression in mammalian cells. Useful selectable markers are well known in the art and include, for example, antibiotic and herbicide resistance genes.

Sources of DNA sequences useful in the present invention include Poly-A RNA from mammalian cells, from which the about 4 kb mRNA encoding human PGHS-2 corresponding to SEQ ID NO. 4 can be derived and used for the synthesis of the corresponding cDNA by methods known to the art. Such sources include the lambda ZAP II (Stratagene) library of size fractionated poly-A RNA isolated from C127 murine fibroblasts treated with serum and cycloheximide as described by M.K. O'Banion et al., J. Biol. Chem., 266, 23261 (1991). Xie et al. obtained mRNA encoding chicken griPGHS as described in PNAS USA, 88, 2692 (1991). Sources of human mRNA encoding griPGHS include RNA from human monocytes treated with interleukin-1 and cycloheximide, in accord with M.K. O'Banion et al., PNAS USA, 89, 4888 (June 1992). Sources of human mRNA encoding PGHS-1 are also well known to the art.

Selectable marker genes encoding enzymes which impart resistance to biocidal compounds are listed in Table 1, below.

**Table 1**

| Selectable Marker Genes | | |
|---|---|---|
| Resistance Gene or Enzyme | Confers Resistance to: | Reference |
| Neomycin phosphotransferase (neo) (see Figure 4). | G-418, neomycin, kanamycin | P.J. Southern et al., J. Mol. Appl. Gen., 1, 327 (1982) |
| | | |
| Hygromycin phosphotransferase (hpt or hyg) | Hygromycin B | Y. Shimizu et al., Mol. Cell Biol., 6, 1074 (1986) |
| | | |
| Dihydrofolate reductase (dhfr) | Methotrexate | W.W. Kwok et al., PNAS USA, 4552 (1986) |
| | | |
| Phosphinothricin acetyltransferase (bar) | Phosphinothricin | M. DeBlock et al., EMBO J., 6, 2513 (1987) |
| | | |
| 2,2-Dichloropropionic acid dehalogenase | 2,2-Dichloropropionic acid (Dalapon) | V. Buchanan-Wollaston et al., J. Cell. Biochem., Supp. 13D, 330 (1989) |
| | | |
| Acetohydroxyacid synthase | Sufonylurea, imidazolinone and triazolopyrimidine herbicides | P.C. Anderson et al. (U.S. Patent No. 4,761,373); G.W. Haughn et al., Mol. Gen. Genet., 211, 266 (1988) |
| | | |
| 5-Enolpyruvylshikimate-phosphate synthase (aroA) | Glyphosate | L. Comai et al., Nature, 317, 741 (1985) |
| | | |
| Haloarylnitrilase | Bromoxynil | D.M. Stalker et al., published PCT appln. WO87/04181 |
| | | |
| Acetyl-coenzyme A carboxylase | Sethoxydim, haloxyfop | W.B. Parker et al., Plant Physiol., 92, 1220 (1990) |
| | | |
| Dihydropteroate synthase (sul I) | Sulfonamide herbicides | F. Guerineau et al., Plant Molec. Biol., 15, 127 (1990) |
| | | |
| 32 kD photosystem II polypeptide (psbA) | Triazine herbicides | J. Hirschberg et al., Science, 222, 1346 (1983) |
| | | |
| Anthranilate synthase | 5-Methyltryptophan | K. Hibberd et al., (U.S. Patent No. 4,581,847) |
| | | |
| Dihydrodipicolinic acid synthase (dap A) | Aminoethyl cysteine | K. Glassman et al., published PCT application No. WO89/11789 |

Reporter genes are used for identifying potentially transformed cells and for evaluating the functionality of regulatory sequences. Reporter genes which encode for easily assayable marker proteins are well known in the art. In general, a reporter gene is a gene which is not present in or expressed by the recipient organism or tissue and which encodes a protein whose expression is manifested by some easily detectable property, e.g., enzymatic activity. Preferred genes includes the chloramphenicol acetyl transferase gene (cat) from Tn9 of E. coli, the beta-glucuronidase gene (gus) of the uidA locus of E. coli, and the luciferase gene from firefly Photinus pyralis. Expression of the reporter gene is assayed at a suitable time after the DNA has been introduced into the recipient cells.

Other elements such as introns, enhancers, polyadenylation sequences and the like, may also be a part of the recombinant DNA sequence. Such elements may or may not be necessary for the function of the DNA, but may provide improved expression of the DNA by affecting transcription, stability of the mRNA, or the like. Such elements may be included in the DNA as desired to obtain the optimal performance of the transforming DNA in the cell.

The recombinant DNA sequence can be readily introduced into the target cells by transfection with an expression vector, such as a viral expression vector, comprising cDNA encoding griPGHS or PGHS-1 by the modified calcium phosphate precipitation procedure of C. Chen et al., Mol. Cell. Biol, 7, 2745 (1987). Transfection can also be accomplished by other methods, including lipofection, using commercially available kits, e.g., provided by BRL.

The invention will be further described by reference to the following detailed examples.

### Example 1. Isolation, Cloning and Sequencing of Murine PGHS-2 Gene

A. *Cells and Cell Cultures* -- C127 mouse fibroblasts were obtained from Peter Howley (NIH) and propagated in high glucose Dulbecco's modified Eagle's medium supplemented with 10% fetal bovine serum (HyClone Laboratories) without antibiotics. See, D.R. Lowy et al., J. Virol., 26, 291 (1978). Cultures were monitored for mycoplasma contamination by Hoechst 33258 staining in accord with the procedure of T.R. Chen, Exp. Cell Res., 104, 255 (1977).

Exponentially growing, subconfluent (60-80%) cell monolayers (35-mm plates) were labeled in Dulbecco's modified Eagle's medium without methionine (GIBCO) plus 200 µCi/ml Tran³⁵S-label (>1,000 Ci/mmol; ICN) for 15 or 30 min. In some cases, fresh fetal calf serum (10%) was present during the labeling period. Monolayers were rinsed twice with ice-cold Dulbecco's modified Eagle's medium (DMEM) with methionine prior to lysis in 200 µl of A8 buffer (9.5 M urea, 2% (w/v) Nonidet P-40, 2% (w/v) ampholines (LKB, 1.6% pH range 5-8, 04.% pH range 3.5-10), 5% (w/v) 2-mercaptoethanol). Incorporation of label into proteins was determined by trichloroacetic acid precipitation. Dexamethasone (Sigma) was freshly prepared in phosphate-buffered saline (PBS) (stock concentrations based on molar extinction coefficient of 1.5 X 10⁴ liters/mol/cm at 250 nn) and added to 1 µM. The calcium ionophore A23187 (Calbiochem) was used at a concentration of 5 µM from a 2.5 mM stock in ethanol. Cychoheximide (Sigma) was used at a concentration of 25 µM from a 100 X stock in water. This level inhibited protein synthesis by >97% within 15 min. Control cultures received appropriate amounts of solvents.

Cyclooxygenase activity was determined in the culture medium by addition of exogenous arachidonic acid substrate (30 µM for 15 min. at 37°C) followed by conversion of the prostaglandin E₂ product to a methyl oximate form. This bicyclic derivative was then quantitated by radioimmunoassay (kit from Amersham Corp.).

B. *RNA Preparation --* Total RNA was isolated from 15-cm plates using guanidinium isothiocyanate lysis followed by centrifugation through a cesium chloride cushion (J.M. Chirgwin et al., Biochemistry, 18, 5294 (1979)). Poly(A) RNA was prepared by two passes through oligo(dT)-cellulose columns, as disclosed by H. Aviv et al., PNAS USA, 69, 1408 (1972). RNAs were quantitated by absorbance measurements at 260 nm.

C. *cDNA Synthesis* Fifty µg of poly-A enriched RNA from C127 cells treated for 2.5 hr. with serum and cycloheximide (25 µM) were then fractionated on a 10-30% sucrose gradient in the presence of 10 mM CH₃HgOH as disclosed by J. Sambrook et al., cited above. Every other fraction was assayed for the presence of the 4kb mRNA by Northern blot analysis using the 1.6 kb 5' end of the ovine PGHS cDNA (obtained from Oxford Biomedical Research, Inc.) labeled by random priming. RNA samples and molecular weight markers (3 µg; Bethesda Research Laboratories RNA ladder) were subjected to formaldehyde-agarose gel electrophoresis (J. Sambrook et al., Molecular Cloning, cited above at pages 7.30-7.32) and then blotted to nylon membranes (Duralon, Stratagene) by overnight capillary transfer in 10 X SSC (1 X SSC is 0.15 M NaCl, 0.015 M sodium citrate).

cDNAs were prepared from fractions enriched in the 4-kb mRNA by oligo(dT) priming ((U. Gubler et al., Gene (Amst.), 25, 263 (1988)) kit from Stratagene) and ligated into λ-ZAP II ((J.M. Short et al., Nucleic Acids Res., 16, 7583 (1988)) Stratagene). Two hundred fifty thousand plaques were screened with the ovine PGHS probe under conditions of reduced stringency (30% formamide, hybridization temperature reduced to 42°C, filters washed in 2 X SSC + 0.1% SDS at 55°C). Double-strand dideoxy termination sequencing of Exo III nested deletion subclones was carried out in both directions using T7 DNA polymerase. See, Heinikoff, Gene., 28, 351 (1984); G. Del Sal et al., Bio-Techniques, 7, 514 (1989).

D. In *Vitro Transcription, In Vitro Translation, Immunoprecipitation, and Primer Extension* -- One µg of cDNA in a Bluescript vector (Stratagene) was linearized at the 3' end with Xho I and transcribed with T3 RNA polymerase in a reaction containing the capping reagent m⁷G(5')ppp(5')G (kit from Stratagene). After purification, one-fifth of the transcribed RNA and 2.5 µg of poly-A RNA purified as described above, from cycloheximide and serum-treated C127 cells were translated in separate *in vitro* reactions containing ³⁵S-methionine as described by the manufacturer (Promega) except that the RNAs were preincubated with 3.5 mM CH₃HgOH for 10 min at room temperature. Reactions were diluted in a modified RIPA buffer and precipitated with polyclonal anti-PGHS serum (Oxford Biomedical Research, Inc.) or first precleared by incubating for 30 min with 50 µl/ml protein A-Sepharose (Pharmacia LKB Biotechnology Inc.; 50% (v/v)). 0.01 volume of antiserum or normal rabbit serum was added to the lysate and allowed to incubate for 2 hr at 4°C prior to precipitation with protein A-Sepharose. The pelleted beads were washed four times with immunoprecipitation buffer and then resuspended in Laemmli lysis buffer for 30 min at room temperature. The immunoprecipitated products were resolved by standard 10% SDS-PAGE and visualized by fluorography.

For primer extension analysis two µg of poly-A RNA from C127 cells treated for 2 hr with serum and cycloheximide was reverse-transcribed with M-MuLV reverse transcriptase (BRL) as described by C.C. Baker et al., EMBO J., 6, 1027 (1987), using a ³²P-end-labeled oligonucleotide complementary to nucleotide (nt) 55-75 of the sequenced 4.1 kb cDNA. Reaction products were electrophoresed on a standard sequencing gel in parallel with an ³⁵S-labeled dideoxy sequencing reaction of the cDNA in its Bluescript vector using the same primer.

E. *cDNA Expression and PGE*_{*2*} *Determination* -- In order to determine whether the 4.1 kb mRNA encodes a protein with cyclooxygenase activity, the cDNA was inserted into an SV40 late promoter expression vector (SVL, (R. Breatnach et al., Nucleic Acid Res., 11, 7119 (1983))). As reported by D. L. DeWitt et al., J. Biol. Chem., 265, 5192 (1990), COS cells have little or no autologous cyclooxygenase activity. Therefore, these cells were transfected with 2.5 or 5 µg of either the vector alone or the vector containing the 4.1 kb cDNA. Two-dimensional gel electrophoresis of ³⁵S-labeled proteins from transfected cells showed a protein doublet (72/74 kDa, pl 7.5) in the 4.1 kb cDNA-expressing cells that corresponds exactly to the immunoprecipitated cyclooxygenase protein doublet observed in C127 mouse fibroblasts whose synthesis is increased by growth factors and decreased by glucocorticoid hormones.

Transfected cells were also assayed for cyclooxygenase activity. COS cells expressing the 4.1 kb cDNA produced nearly two orders of magnitude more prostaglandin E₂ than control cells (Table 2). Furthermore, prostaglandin production increased with the amount of transfected DNA. These results unequivocally demonstrate that the 4.1 kb mRNA encodes an active cyclooxygenase which was designated "glucocorticoid-regulated inflammatory PGHS (griPGHS).

**Table 2.**

| Expression of the 4.1 kb cDNA in COS cells leads to prostaglandin synthesis. Subconfluent COS A.2 cells in duplicate 60 mm plates were transfected with the indicated amounts of expression vector alone (SVL) or the expression vector containing the 4.1 kb cDNA (SVL-4.1) and assayed for PGE₂ production 2 days later. | | |
|---|---|---|
| DNA | Amount | pg PGE₂/µg protein |
| None | - | 0.56, 0.58, 0.51, 0.50 |
| SVL | 2.5 µg | 0.55, 0.68 |
| SVL | 5.0 µg | 0.63, 0.65 |
| SVL-4.1 | 2.5 µg | 14.8, 24.6 |
| SVL-4.1 | 5.0 pg | 63.8, 42.4 |

For PGE₂ production assays, cells were rinsed once with prewarmed DMEM, and then 1 ml of DMEM containing 30 µM arachidonic acid was added. After 10 or 15 min, the supernatants were collected, clarified by brief centrifugation, and assayed for PGE₂ by radio-immunoassay after conversion to the methyl-oximated form (kit from Amersham). Monolayers were solubilized in 0.5 N NaOH, neutralized with 1N HCl, and clarified by centrifugation prior to protein concentration determination.

F. *Northern Blot Analysis* -- Poly-A enriched RNAs (2.5 µg) from C127 cells were fractionated by formaldehyde-agarose gel electrophoresis and transferred to a membrane (Duralon, Stratagene). Hybridization was carried out as previously described by M.K. O'Banion et al, J. Virol., 65, 3481 (1991), using the 5' 1.2 kb EcoR1 fragment of the 4.1 kb cDNA labeled with ³²P by random priming as disclosed by A.P. Feinberg et al., Anal. Biochem., 132, 6 (1983). The membrane was later rehybridized with a similarly labeled portion (1.6 kb 5' end) of the 2.8 kb ovine PGHS cDNA (Oxford Biomedical Research, Inc.), and an end-labeled 40-mer complimentary to β-tubulin (Oncor). RNA molecular weight markers (BRL) were visualized by ethidium bromide staining. A similar analysis was performed on total RNA (5 µg/lane) isolated from human monocytes by the guanidinium-acid-phenol extraction method of P. Chomezynski et al., Anal. Biochem., 162, 156 (1987).

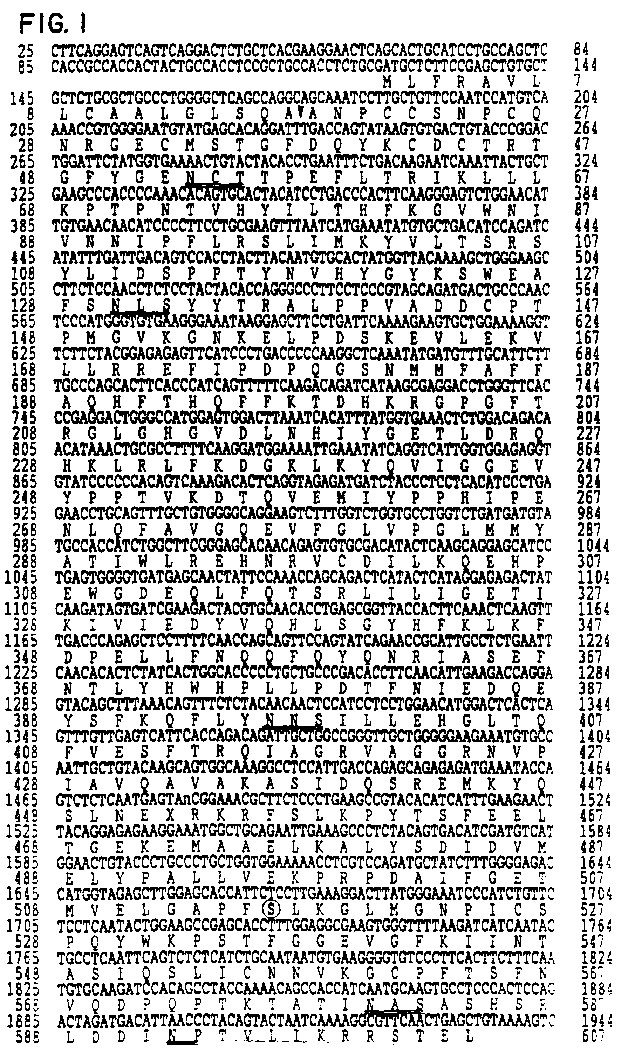

G. *Results* -- A directionally cloned cDNA library was constructed in lambda ZAP II from sucrose gradient fractions enriched in the 4 kb mRNA and screened with a radiolabeled portion of the 2.8 kb PGHS cDNA under conditions of lowered stringency. Several positive plaques were isolated and analyzed. One about 4.1 kb in length was fully sequenced. This clone encodes a 70 kDa protein specifically precipitated by anticyclooxygenase serum, which migrates identically with the immunoprecipitated protein product from *in vitro* translated poly A-mRNA. Primer extension analysis, using a 20-mer starting at nt 75 of the sequence, indicated that transcription starts 24 bases upstream of the cDNA clone. Comparison of the 4.1 kb sequence (Fig. 1) with that of the previously cloned 2.8 kb PGHS cDNA from mice (which is very similar to that cloned from sheep and human tissues), revealed a single open reading frame with 64% amino acid identity to the protein encoded by the 2.8 kb PGHS cDNA. The deduced protein sequences are colinear except that the 4.1 kb cDNA has a shorter amino-terminus and longer carboxy-terminus. The full sequence has been deposited in GenBank, accession number M88242.

Three of four potential N-glycosylation sites are conserved between the two molecules and there is particularly high similarity in the regions surrounding a putative axial heme-binding domain (amino acids 273-342) and the region around the presumed aspirin modified-serine⁵¹⁶ (amino acids 504-550). By far the largest difference in the two cDNAs is the presence of a 2.1 kb 3' untranslated region in the 4.1 kb cDNA. This region is rich in 5'-AUUUA-3' motifs that are associated with the decreased stability of many cytokine and protooncogene mRNAs. The presence of these motifs is consistent with the profound superinducibility of the 4.1 kb mRNA by cycloheximide, which is not observed for the 2.8 kb mRNA.

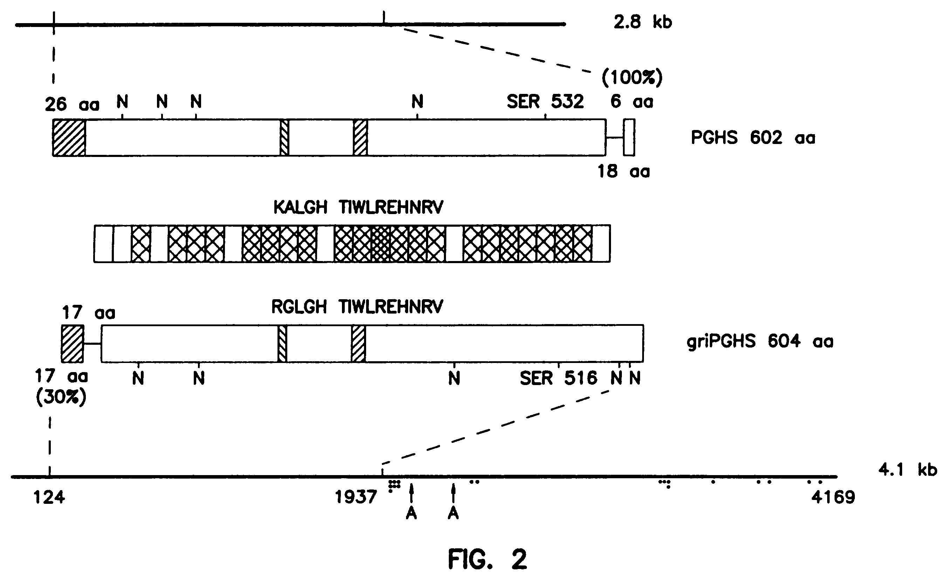

Figure 2 schematically compares cDNA and protein sequences for the murine 2.8 and 4.1 kb mRNA-encoded cyclooxygenases. cDNA structures for the 4.1 kb cDNA cloned from murine C127 cells and the murine 2.8 kb cDNA (D.L. Dewitt et al., J. Biol. Chem., 265, 5192 (1990)) are drawn as the thick lines at top and bottom. The numbering of the 4.1 kb cDNA is based on primer extension data. Since the 5' end of the 2.8 kb mouse mRNA has not been determined, no numbers have been assigned to the translation start and stop sites. Alternative polyadenylation sites established from other cDNA clones are indicated with "A" and the 5'-AUUUnA-3' motifs are identified by dots underneath the sequence. These motifs are not found in the 2.8 kb cDNA. Deduced protein sequences are drawn colinearly with gaps (17 aa at the amino-terminal end of the 4.1 kb mRNA product, and 18 aa at the carboxy-terminal end of the 2.8 kb mRNA product) indicated by connecting lines. The 26 amino acid (aa) leader sequence for the 2.8 kb PGHS is indicated. Although its extent has not been precisely defined, a shorter, nonhomologuous leader appears to exist for griPGHS with a mature N-terminal end at amino acid 18. The positions of potential N-glycosylation sites (NXS/T, "N") and the conserved aspirin modified serines are noted on each molecule. The hatched areas near the center of each molecule denote presumed axial (TIWLREHNRV (SEQ ID NO:7), identical between the two molecules) and distal (KALGH (SEQ ID NO:8) / RGLGH (SEQ ID NO:9)) heme-binding sites as suggested by DeWitt et al., cited above. The bar in the middle of the figure represents the similarities between the two mouse PGHS proteins (omitting the nonconserved N- and C-termini) as the percentage of identical residues for groups of 20 amino acids with increasing shading indicating 40-55% (no shading), 60-75%, 80-95%, and 100% identity. The overall identity is 64% and with conservative changes the similarity index is 79%.

### Example 2. Expression of griPGHS in Human Monocytes

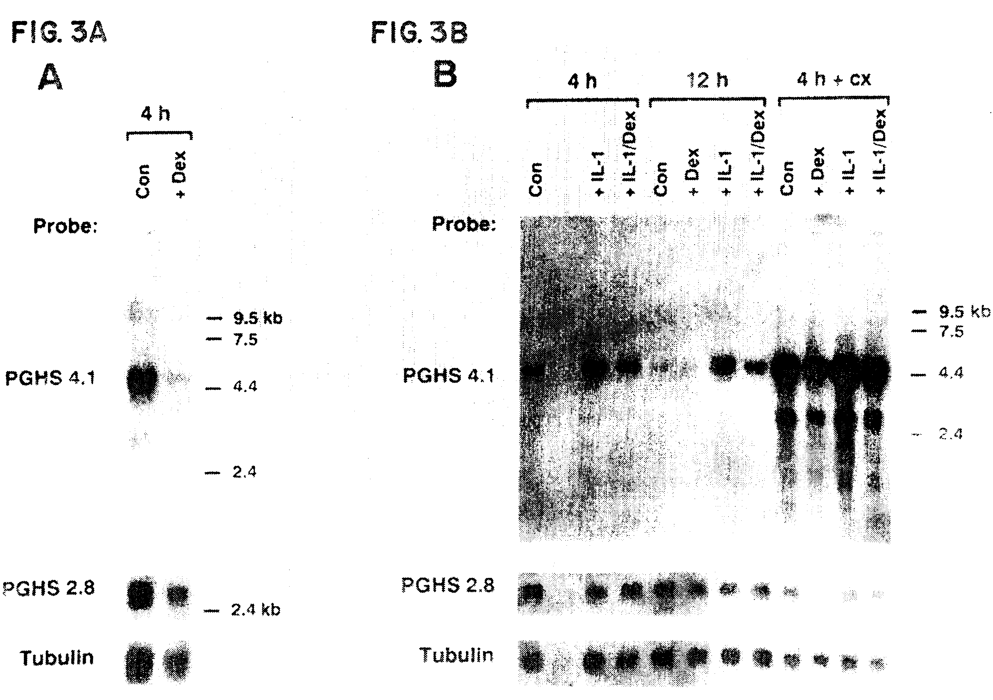

Adherent human monocytes isolated from healthy donors as described by N.J. Roberts et al., J. Immunol., 121, 1052 (1978), were suspended in M199 medium without serum at 1 x 10⁶ cells/ml. One ml aliquots in 5 ml polypropylene tubes were incubated with loosened caps in 5% CO₂ at 37°C with occasional shaking. To derive the autoradiograph shown in Figure 3, Panel A, monocytes were incubated for 4 hr in the presence or absence of dexamethasone (1 µM; Sigma) prior to total RNA isolation by the procedure of P. Chomczynski et al., cited above. Five µg RNA was subjected to Northern blot analysis as described by M.K. O'Banion et al., J. Biol. Chem., 34, 23261 (1991) with the indicated probes labeled by random priming (kit from Boehringer-Mannheim) to a specific activity > 1 x10⁹ cpm/µg. To derive the autoradiograph shown in Figure 3, Panel B, monocytes were treated with dexamethasone (1 µM), IL-1β (10 half-maximal units, Collaborative Research), or both for the indicated times prior to RNA isolation. Cycloheximide (25 µM; Sigma) was added to one set of incubations 15 min prior to the addition of cytokine or hormone.

Figure 3 depicts Northern blots of total monocyte RNA and demonstrates that a 4.8-kb mRNA species is detected with the mouse griPGHS 4.1-kb probe. When normalized to the hybridization signal for β-tubulin, griPGHS mRNA levels are down-regulated by dexamethasone at 4 hr (5-fold in this example), while the level of the 2.8-kb PGHS mRNA is not affected. In this experiment, the level of accumulated PGE₂ in the supernatant after 4 hr of incubation was reduced by dexamethasone from 122.5 to 52.5 pg per 10⁴ monocytes. In another experiment, monocytes treated with IL-1β showed increased levels of griPGHS mRNA at 4 hr (2.5-fold relative to control) and 12 hr (14-fold) (Figure 3). These increases were significantly blunted when dexamethasone was present. Furthermore, the IL-1β induction and dexamethasone repression of griPGHS mRNA abundance occurred in the presence of cycloheximide, where superinduction of the 4.8-kb mRNA was clearly evident (Figure 3). In contrast, levels of the 2.8-kb mRNA were not significantly altered relative to β-tubulin by IL-1β, dexamethasone, or cycloheximide treatment.

### Example 3. Drug Assays Using griPGHS Transfectants

A. *Expression vector construction* -- Following the methodology of J.M. Short et al., Nucleic Acids Res., 16, 7583 (1988), the 4.1 griPGHS cDNA clone was excised *in vivo* from the lambda ZAP II vector and the resulting griPGHS-Bluescript construct isolated on ampicillin plates. griPGHS was prepared for directional subcloning into the pRC/CMV expression vector (Invitrogen) by digestion with Acc I, Klenow fill-in, and digestion with Not I. This fragment, extending from the Not I site 50 bases upstream of the cDNA end to nt 1947 of the cDNA, was isolated by gel electrophoresis and contains the full-coding region truncated immediately before any 5'-AUUUA-3' mRNA destabilizing regions. The pRc/CMV vector DNA was digested with Xba I, filled in with Klenow, then digested with Not I. It was further prepared by calf intestinal alkaline phosphatase treatment. Ligated pRc/CMV-griPGHS recombinants were isolated from ampicillin plates following transformation into competent DH5α cells (Library Efficiency; Life Science Technologies), and were confirmed by restriction analysis of DNA mini-preps. The construct is illustrated in Figure 4.

B. *Transfection and establishment of stable cell lines* -- Sixty-mm plates of subconfluent COS A2 cells, which contain little or no autologous cyclooxygenase activity, were transfected with 1 or 2.5 µg of purified griPGHS-pRC/CMV, or the vector alone, by lipofection for 23 hr following the manufacturer's directions (Life Science Technologies). After 2 days of growth in normal media (DMEM + 10% fetal bovine serum), transfected cells were switched to media containing 800 µg/ml of Geneticin (G418, active component 657 µg/ml; Life Science Technologies), a concentration previously found to be toxic for COS cells. The media was changed every 3 days, and after 2 weeks many individual colonies were observed in the dishes transfected with either recombinant or vector alone, but not in the dishes with no transfected DNA. A total of 36 griPGHS pRc/CMV-transfected and 12 vector-transfected colonies were isolated using cloning cylinders. The majority of these survived continued selection in 800 µg/ml G418 during clonal line expansion. Established cultures are maintained in DMEM + 10% fetal bovine serum with 400 µg/ml G418.

C. *Drug Studies* -- Prostaglandin assays were carried out as described above. For drug studies, cells were exposed to various concentrations of drugs for 30 min in serum-free DMEM and arachidonic acid was added directly from a 25x stock in DMEM. Supernatants were harvested 15 min later. Controls consisted of no drugs and wells treated with maximal concentrations of drug vehicles (1% methanol or ethanol). Drugs were obtained from Sigma and prepared as 200 mM stock solutions (acetaminophen and ibuprofen in methanol, indomethacin in ethanol, and naproxen in water).

### D. Results

1. Expression vector choice -- The pRC/CMV eukaryotic expression vector (Fig. 4) provides several distinct advantages for our purpose. In addition to the ease of selection in both bacterial and eukayotic hosts, expression of the present cloned cDNA is driven by a strong CMV promoter. The vector also provides a poly-A signal that is necessary since the present construct does not contain griPGHS 3' untranslated sequences (it ends 12 base pairs (bp) from the translation termination codon). The removal of these sequences is important since *in vivo* they provide signals (5'-AUUUA-3') for rapid mRNA degradation. Finally, the vector is well suited for use in COS cells which have little or no autologous cyclooxygenase activity.

2. Cell line characterization -- Of the 36 griPGHS-pRc/CMV- and 12 vector alone-cloned neomycin resistant colonies, 29 and 9, respectively, were tested for PGE₂ production. In all cases, vector-alone transfectants produced less than 8 pg of PGE₂ per assay (number reflects the amount of PGE₂ secreted after 10 or 15 min in 20 µl of collected media), whereas the griPGHS transfected clones showed a wide range of prostaglandin production. Of these, eleven prostaglandin-producing and 2 vector-alone containing clones were further expanded and retested.

The amount of PGE₂ secreted by the clones harboring the griPGHS construct varied from 10.6 to 72.2 pg/µg cell protein (Table 3).

**Table 3.**

| **PGE**_{**2**} **production by various cell lines.** | |
|---|---|
| Cell Line | pg PGE₂/µg cell protein |
| A2 | 4.4 |
| A5 | 1.9 |
| E1 | 16.7 |
| E7 | 23.6 |
| E8 | 46.8 |
| E9 | 30.5 |
| E11 | 34.2 |
| F3 | 40.0 |
| F4 | 10.6 |
| F6 | 12.2 |
| F8 | 72.1 |
| F14 | 3.5** |
| F15 | 16.8 |

The values in column 2 represent the amount of prostaglandin secreted during a 10 min exposure to 30 µM arachidonic acid and are normalized to total recovered cellular protein. Cell lines A2 and A5 contain the vector alone and the remaining cells were transfected with griPGHS-pRc/CMV. Note that only one (F14, marked by double asterisk) showed no increase PGE₂ production over cells harboring the vector alone.

Each of these lines was expanded for cyropreservation and one (E9), chosen for ease of culturing and its significant PGE₂ production, was used in further studies. A sample of this cell line has been deposited in the American Type Culture Collection, Rockville, MD, U.S.A. under the provisions of the Budapest Treaty and assigned accession number ATCC 11119.

3. Stability of PGE₂ production -- Stable expression of cyclooxygenase activity in the E9 cell line was tested by comparing PGE₂ production over at least 5 passages of the cell line. After 6 weeks, these cells were still producing high levels of PGE₂. Although the numbers are not directly comparable, since cell numbers were not normalized by protein determination in all cases, the amount of PGE₂ secreted by E9 cells in this standard assay ranged from 35 pg to 90 pg (per 20 µl assayed media). Furthermore, within an experiment, E9 cells showed very consistent levels of PGE₂ production from well to well. For example, for 12 tested supernatants, PGE₂ levels were 48.4 ± 3.5 pg/20 µl (mean ± SEM).

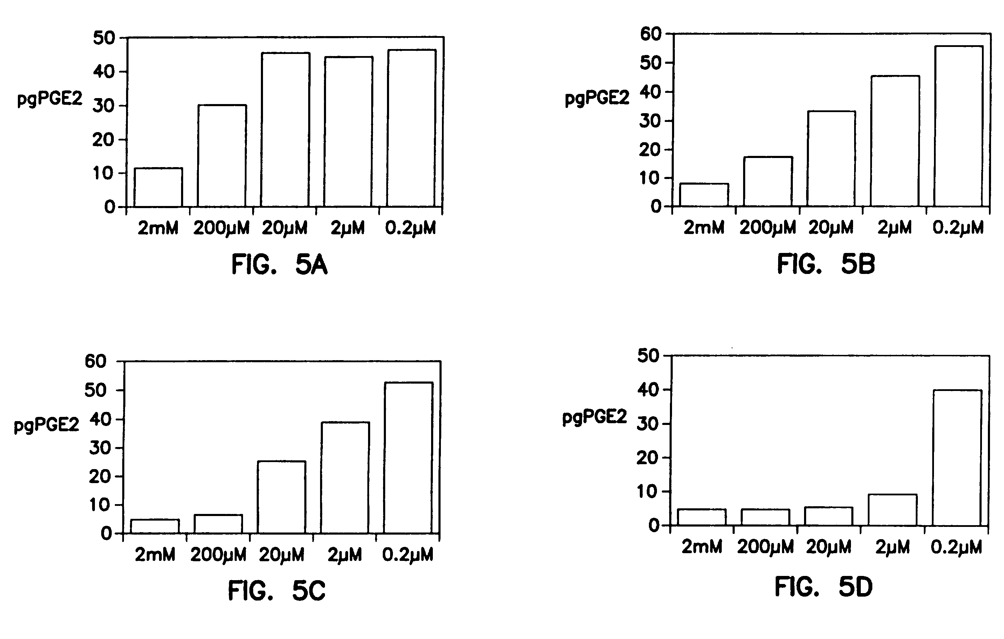

4. Drug studies -- To illustrate the utility of our cell lines in drug testing, we exposed duplicate wells of the E9 cells to a range of doses (0.2 µM - 2 mM) of four non-steroidal anti-inflammatory drugs: acetaminophen, ibuprofen, naproxen, and indomethacin. Cells were placed in serum-free medium with the drugs for 30 min prior to a 15 min exposure to arachidonic acid (added directly to the media). Synthesized PGE₂ was then quantitated from the supernatants by our standard radioimmunoassay. Results, shown in Fig. 5, reveal specific dose-response curves for each drug with indomethacin showing the most and acetaminophen the least potency against griPGHS activity. The maximal inhibition in each case (except for acetaminophen where 2 mM was apparently not sufficient for full inhibition) was similar to that seen for COS cells harboring the vector alone (3-8 pg). Low doses of each drug gave levels corresponding to the untreated control values which averaged at 48.4 pg. Note that controls run both with and without 1% drug vehicle (methanol or ethanol; comparable to exposure in the 2 mM drug conditions) showed no differences in PGE₂ production.

### Example 4. Preparation of Microsomal Extracts and In Vitro Testing of Cyclooxygenase Activity

Microsomal extracts and measurements of cellular cyclooxygenase activity are performed essentially as described by A. Raz et al., J. Biol. Chem., 263, 3022 (1988); and PNAS USA, 86, 1657 (1989). Cells are rinsed once with ice-cold PBS (pH=7.4), scraped from dishes with a plastic disposable scraper (Gibco), transferred to 1.5 ml microfuge tubes with ice-cold PBS, and pelleted by centrifugation (8 minutes at 800xg). The supernatants are removed and the cell pellets rinsed with additional PBS. Cell pellets can be stored at -70°C at this point.

To prepare extracts, the pellets are resuspended in solubilization buffer (50 mM Tris, 1mM diethyldithiocarbamic acid (sodium salt), 10 mM EDTA, 1% (v/v) Tween-20 and 0.2 mg/ml α₂-macroglobulin, pH=8.0), followed by sonication (5 x 10 sec bursts, low power setting). Extracts are clarified by centrifugation at 4°C (20 minutes at 16,000xg). Aliquots are taken for protein determination, and 50 µl aliquots are diluted to 500 µl with a solution containing 100 mM NaCl, 20 mM sodium borate, 1.5 mM EDTA, 1.5 mM EGTA, 0.3 mM PMSF, 10 mM NEM, 0.5% BSA, 0.5% Triton X-100, 1mM epinephrine and 1mM phenol (pH=9.0).

Reactions are initiated by the addition of arachidonic acid in the above buffer to 100 µM of microsomal extract and incubated for 30 minutes at 37°C. The PGE₂ formed is measured by RIA after quantitative conversion to the methyl oximated form as described by the RIA kit manufacturer (Amersham). To test the effects of non-steroidal anti-inflammatory compounds, different doses of drugs are added 5 min prior to initiating the reaction with arachidonic acid.

### Example 5. Generation of Human PGHS-1 and Human PGHS-2 cDNA Clones

RNA was isolated from a human fibroblast cell line (W138) treated with serum and cycloheximide for 4 hr. Total RNA isolation was done by guanidinium lysis followed by CsCl cushion centrifugation (J.M. Chirgwin et al., Biochem., 18, 5294 (1977)). Polymerase chain reaction (PCR) primers specific for the human PGHS-1 and PGHS-2 sequences were engineered to amplify the coding regions of either one transcript or the other (Table 4). The 5' end primers contained a Hind III restriction site and the 3' end primers contained a Not I restriction site for subsequent cloning. Reverse transcriptase polymerase chain reactions (RT-PCR) carried out as described by E. S. Kawasaki, in PCR Protocols: A Guide to Methods and Applications, M.A. Innis et al., eds., Academic Press, NY (1990), using the specific primers generated PCR products about 2kb in size.

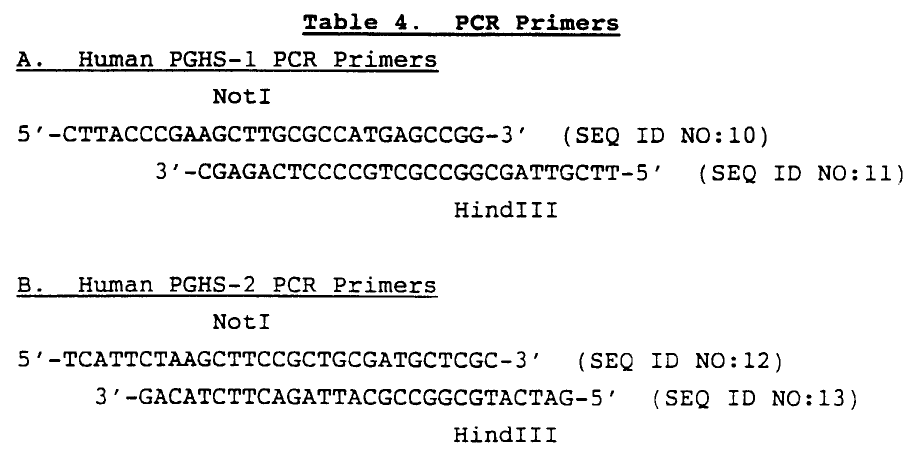

### Example 6. Determination of Sequences and Generation of Plasmid Constructs for Transfection

Following purification and digestion with HindIII and NotI, the two PCR products were each ligated into pRC/CMV vectors (Invitrogen) (see Figure 4). Ligated pRC/CMV-PGHS recombinant plasmids were isolated from ampicillin plates following transformation into competent DH5a cells (BRL). Clones were screened by for the presence of PGHS inserts by restriction mapping.

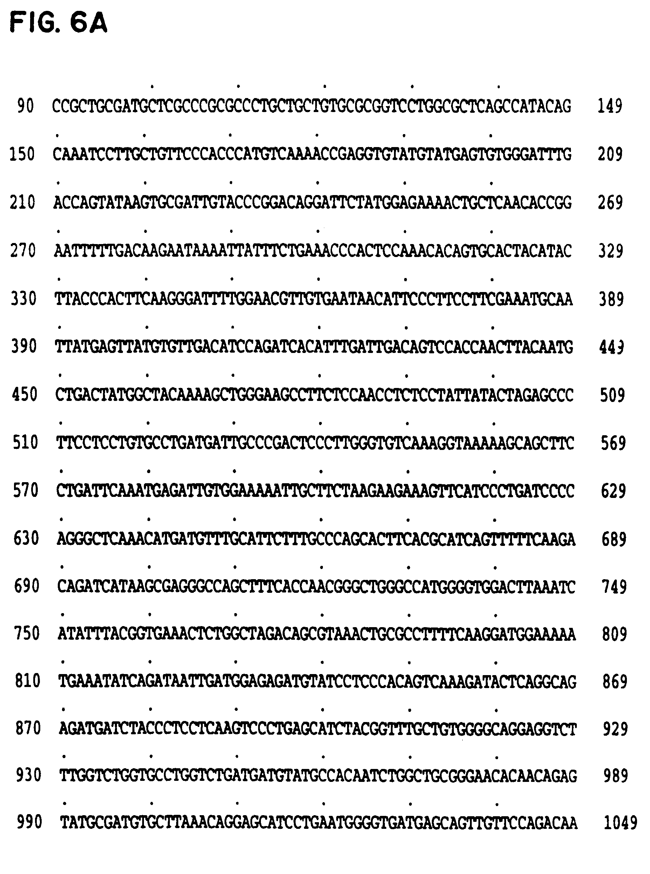

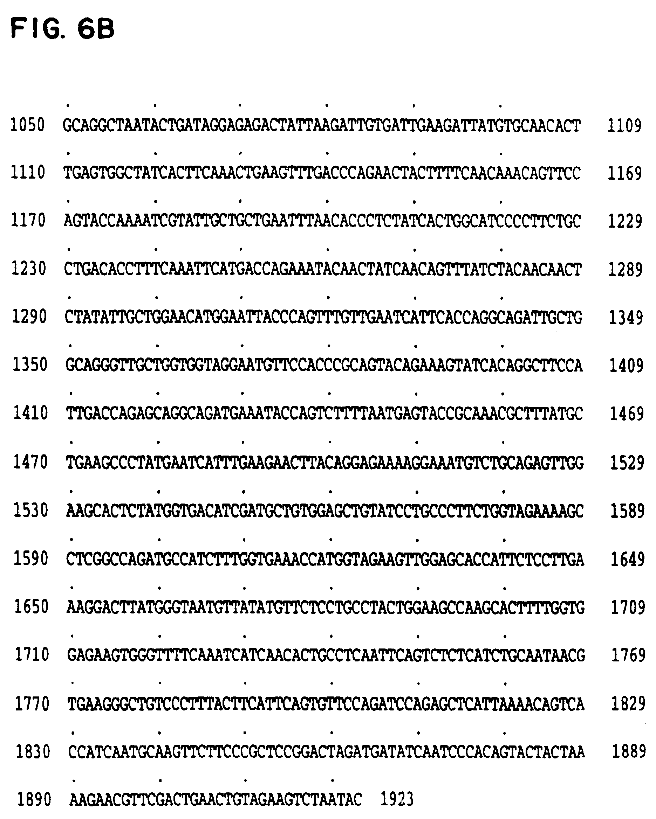

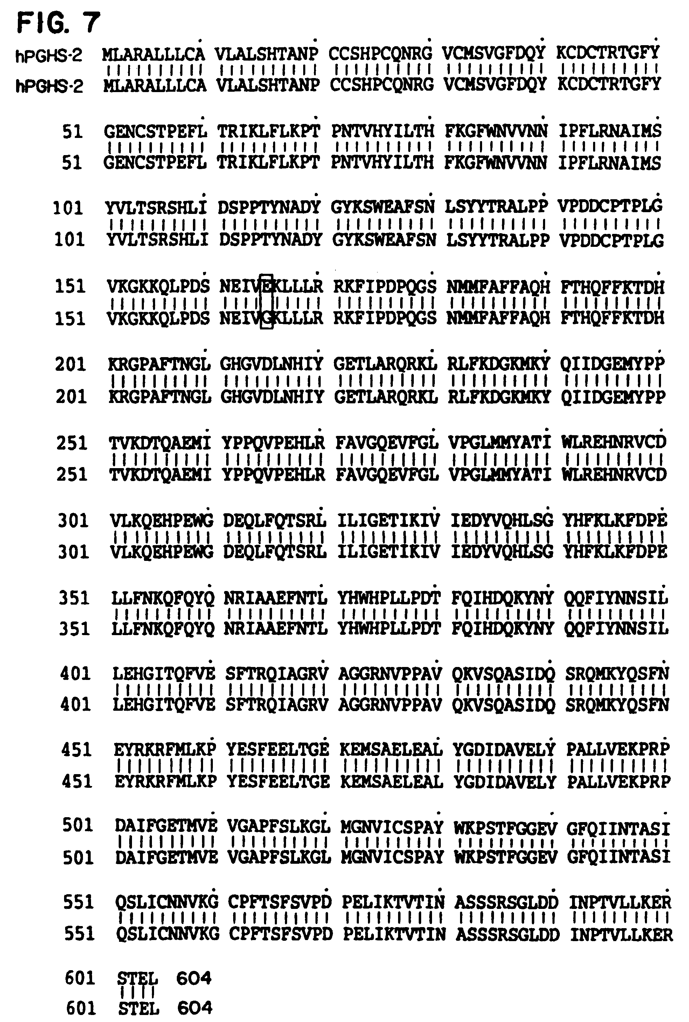

Three PGHS-2 clones were sequenced in both directions on an Applied Biosystems automated sequencer Model #373A. The clone comprising the PGHS-2 gene sequence depicted in Figure 6 was selected for transfection. This sequence differs from the human PGHS-2 sequence disclosed by Hla and Neilson, PNAS, 89, 7384 (1992), due to a glutamic acid (E) rather than a glycine (w) at amino acid position 165 of the PGHS-2 gene product (Figure 7). The sequence for the PGHS-2 gene was confirmed by establishing the identity of the sequences of two other hPGHS-2 clones obtained from separate PCR runs, which demonstrates that the difference observed is not a PCR artifact. Furthermore, as shown in Figure 1, mouse PGHS-2 also has a glutamic acid at this position. PGHS-1 clones were similarly screened and the sequences of the PGHS-1 gene and enzyme confirmed to be identical to that shown in Figure 2 (SEQ ID NO:6) in C. Tokoyama et al., Biochem. Biophys. Res. Commun., 165, 888 (1989); see also, T. Hla, Prostaglandins, 32, 829 (1986).

### Example 7. Generation of Stably Transfected Mammalian Cell Lines

Sixty-mm plates of 50% confluent COS-A2 (monkey-kidney) cells, which contain little or no cyclooxygenase activity were transfected with 1-2.5 µg of purified pRC/CMV;hPGHS-2 plasmid, pRC/CMV;hPGHS-1 plasmid or the pRC/CMV vector alone by a calcium phosphate precipitation method (Chen et al., Mol. Cell. Biol., 7, 2745 (1987)). Plates were incubated at 35°C, 3% CO₂ for 24 hr in normal media (Dulbecco's Modified Eagle Media (DMEM) + 10% fetal bovine serum). After two rinses with warm DMEM, plates were transferred to 37°C, 5% CO₂ for an additional 24 hr. Selection was then started with normal media containing 800 µg/ml of Geneticin (active component G418, 657 µg/ml, Life Science Technologies), a concentration which is toxic for COS cells. The media was changed every 3 days and after 2 weeks, many individual colonies were observed in the dishes transfected with either recombinant PGHS vector or vector alone, but not in the dishes with no transfected DNA. Twelve to twenty-four colonies from each transfection were isolated using cloning cylinders. The majority of these survived continued G418 selection during clonal cell-line expansion. Established cultures are maintained in DMEM + 10% fetal bovine serum with 400 µg/ml G418.

### Example 8. Testing the G418 Resistant Cell Lines and Confirming the Stable Expression of PGHS-2 and PGHS-1 Activity

Transfected COS cells plated in 12-well plates were grown to near confluence, rinsed twice with warm serum-free media and then covered with 300 µl of 30 µM arachidonic acid (sodium salt; Sigma). After 15 min, supernatants were placed in Eppendorf tubes on ice, clarified by centrifugation at 15,000 x g for 2 min, and assayed for PGE₂ production by immunoassay after conversion to the methyl oximated form (kit from Amersham).

Cell monolayers were solubilized in 0.5 M NaOH and neutralized with 1 M HCl for protein concentration determinations using reagents from BioRad (modified Bradford Assay). Cell lines expressing PGHS activity were further expanded and then frozen down in media with 10% DMSO.

Cell line 4B4 expressing PGHS-2 and cell line H17A5 expressing PGHS-1 were deposited on March 5, 1993 in the American Type Culture Collection, Rockville, MD, USA (cell line 4B4 was assigned ATCC accession number CRL 11284; cell line H17A5 was assigned ATCC CRL 11283).

Levels of PGHS expression in the stably transformed cell lines varied and were much higher for PGHS-1 cell lines in comparison to PGHS-2 cell lines, as shown by the data in Table 5.

**Table 5.**

| **PGE**_{**2**} **Production in Stably Transformed COS Cell Lines** | | | |
|---|---|---|---|
| Human PGHS-1 Cell Lines (pRC/CMV;hPGHS-1) | | Human PGHS-2 Cell Lines (pRC/CMV;hPGHS-2) | |
| Line | Level^{a} | Line | Level^{a} |
| H17A1 | 0.4 | 2A2 | 5.5 |
| H17A3 | 2500 | 2B1 | 4.0 |
| H17A5* | 2500+ | 2B2 | 37.5 |
| H17A6 | 73.5 | 2B3 | 31.6 |
| H17B3 | 145 | 2B5 | 39.6 |
| H17B6 | 1640 | 2B6 | 29.0 |
| H22A2 | 2036 | 4A1 | 36.2 |
| H22A5 | 40.3 | 4A2 | 0.4 |
| H22B2 | 73.5 | 4A3 | 0.6 |
| H22B3 | 568 | 4A4 | 8.2 |
| H22B4 | 9.2 | 4A5 | 9.8 |
| | | 4A6 | 7.2 |
| | | 4B1 | 24.6 |
| | | 4B2 | 4.8 |
| | | 4B3 | 13.1 |
| | | 4B4* | 58.0 |
| | | 4B5 | 10.6 |

| | | | |
|---|---|---|---|
| ^{a} Pg PGE₂/15 min/µg cellular protein; COS-A2 = 0.4; COS-A2 + pRC/CMV vector = 0.4 | | | |

The cell lines have maintained high levels of PGHS expression even after many months of culturing. For example, the cell line 4B4 has been tested 6 times over 5 months and expression has ranged from 50-60 pg PGE₂/µg cellular protein. The exclusive presence of either PGHS-1 or PGHS-2 in the cell lines was confirmed by Northern analyses using hybridization probes that are specific for either PGHS-1 or PGHS-2.

### Example 9. Nonsteroidal Anti-inflammatory Drug (NSAID) Studies on Stable Human PGHS-1 and PGHS-2 Cell Lines

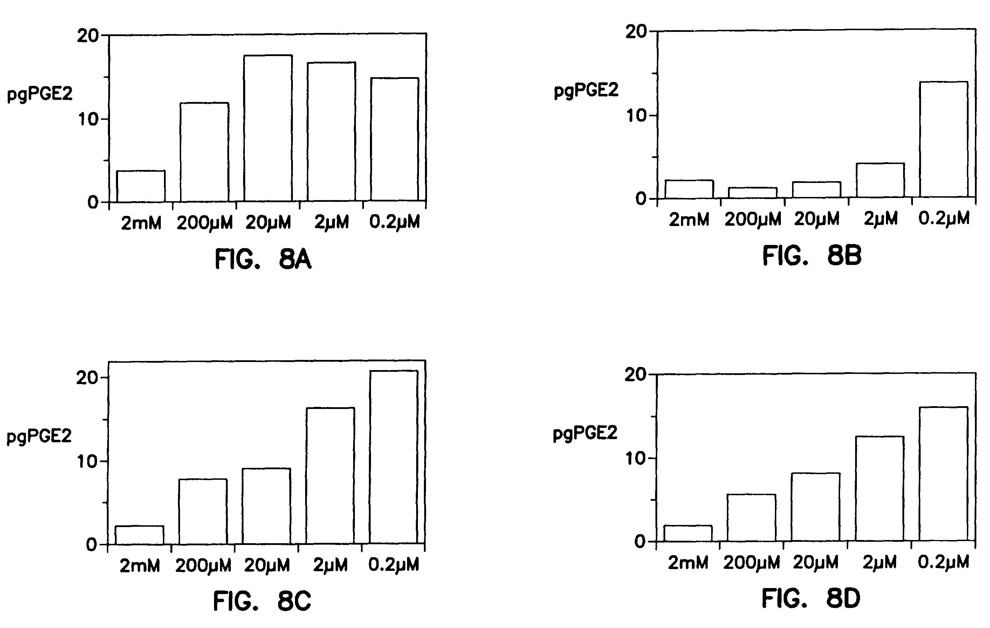

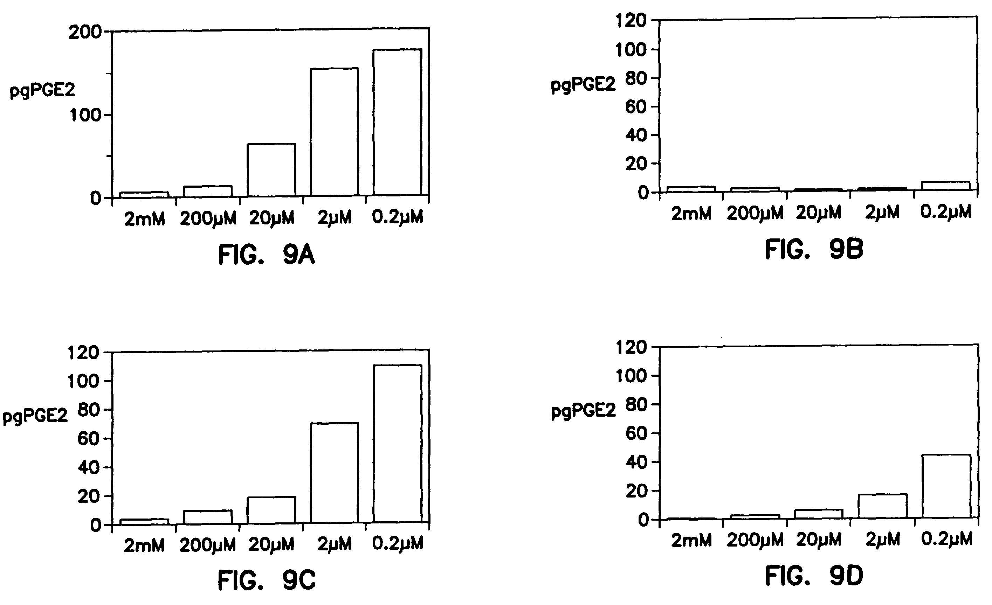

PGHS-1 and PGHS-2 cell lines (including 4B4 and H17A5) were exposed to various concentrations of NSAID for 30 min in serum-free DMEM. Arachidonic acid was added directly from a 25x stock in DMEM and supernatants were harvested 15 min later. Controls consisted of no drug treatment and cells treated with the maximal concentrations of drug vehicles (1% methanol or ethanol). Drugs were obtained from Sigma Chem. Co. and prepared as 200 mM stock solutions (aspirin and ibuprofen in methanol, indomethacin in ethanol, and naproxen in water). Cyclooxygenase activity was determined as described herein above. Distinctly different dose-response curves that were characteristic for either the PGHS-1 or PGHS-2 cell lines were observed. Particularly as shown in Figures 8 and 9 for indomethacin and aspirin, the levels of drug required for inhibition were different for the cells expressing PGHS-1 versus those expressing PGHS-2 (Figures 8-9).

The present invention provides a simple *in vitro* system for the screening of drug actions on both the constitutive and the inflammatory cyclooxygenase, which will be useful for the development of drugs that selectively inhibit inflammation without producing the side effects due to inhibition of constitutive prostaglandin production. Assays can be performed on living mammalian cells, which more closely approximate the effects of a particular serum level of drug in the body, or on microsomal extracts prepared from the cultured cell lines. Studies using microsomal extracts offer the possibility of a more rigorous determination of direct drug/enzyme interactions.

All publications, patents and patent applications are herein incorporated by reference to the same extent as if each individual publication or patent application was specifically and individually indicated to be incorporated by reference.

It will be apparent to one of ordinary skill in the art that many changes and modifications can be made in the invention without departing from the spirit or scope of the appended claims.

## Claims

1. A method of determining the ability of a compound to inhibit prostaglandin synthesis catalyzed by PGHS-2 in mammalian cells comprising:
(a) adding a preselected amount of said compound to a transgenic mammalian cell line in culture medium, which cell line contains chromosomally integrated, recombinant DNA sequence, wherein said DNA expresses human PGHS-2 having an amino acid sequence corresponding to SEQ ID No. 4, and wherein said DNA does not express PGHS-1, and wherein said cell line does not express autologous PGHS-1 or PGHS-2 activity;
(b) adding arachidonic acid to said culture medium;
(c) measuring the level of a PGHS-mediated arachidonic acid metabolite synthesized by cell line; and
(d) comparing said level with the level of said metabolite synthesized by said cell line in the absence of said compound.

2. The method of claim 1 wherein the metabolite is a prostaglandin.

3. A method of determining the ability of a compound to inhibit prostaglandin synthesis catalyzed by PGHS-2, comprising:
(a) preparing a microsomal extract of a transgenic mammalian cell line which contains a chromosomally integrated, recombinant DNA sequence, wherein said DNA sequence expresses human PGHS-2 having an amino acid sequence corresponding to SEQ ID No. 4, and wherein said DNA sequence does not express PGHS-1, and wherein said cell line does not express autologous PGHS-1 or PGHS-2 activity;
(b) forming a buffered aqueous mixture comprising a portion of the microsomal extract and a preselected amount of said compound;
(c) adding arachidonic acid to said mixture;
(d) measuring the amount of a PGHS-mediated arachidonic acid metabolite synthesized in said mixture; and
(e) comparing said amount to the amount of said metabolite synthesized by a second portion of said microsomal extract in the presence of arachidonic acid, but in the absence of said compound.

4. The method of claim 3 wherein said metabolite is a prostaglandin.

5. A method of determining the ability of a compound to inhibit prostaglandin synthesis catalyzed by PGHS-2 or PGHS-1 in mammalian cells comprising:
(a) adding a first preselected amount of said compound to a first transgenic mammalian cell line in culture medium, which cell line contains a chromosomally integrated, recombinant DNA sequence, wherein said DNA sequence expresses human PGHS-2 having an amino acid sequence corresponding to SEQ ID No. 4, and wherein said DNA sequence does not express PGHS-1, and wherein said cell line does not express autologous PGHS-1 or PGHS-2 activity;
(b) adding arachidonic acid to said culture medium;
(c) measuring the level of a PGHS-mediated arachidonic acid metabolite synthesized by said first cell line;
(d) comparing said level with the level of said metabolite synthesized by said first cell line in the absence of said compound;
(e) adding a second preselected amount of said compound to a second transgenic mammalian cell line in culture medium, which cell line contains chromosomally integrated, recombinant DNA sequence, wherein said DNA sequence expresses mammalian PGHS-1, and wherein said DNA sequence does not express any mammalian PGHS-2, and wherein said cell line does not express autologous PGHS-1 or PGHS-2 activity;
(f) adding arachidonic acid to said culture medium;
(g) measuring the level of a PGHS-mediated arachidonic acid metabolite synthesized by said second cell line; and
(h) comparing said level with the level of said metabolite synthesized by said second cell line in the absence of said compound.

6. The method of claim 5 wherein in step (e), the mammalian PGHS-1 is human PGHS-1.

7. The method of claim 5 or claim 6 wherein, in steps (c) and (g), the metabolite is a prostaglandin.

8. The method of any one of claims 5 to 7 wherein, in steps (a) and (e), the transgenic mammalian cell lines are primate cell lines.

9. A method of determining the ability of a compound to inhibit prostaglandin synthesis catalyzed by PGHS-1 or PGHS-2 comprising:
(a) preparing a first microsomal extract of a first transgenic mammalian cell line which contains a chromosomally integrated, recombinant DNA sequence, wherein said DNA sequence expresses human PGHS-2 having an amino acid sequence corresponding to SEQ ID No. 4, and wherein said DNA sequence does not express PGHS-1, and wherein said cell line does not express autologous PGHS-1 or PGHS-2 activity;
(b) forming a first aqueous mixture comprising a portion of the first microsomal extract and a first preselected amount of said compound;
(c) adding arachidonic acid to said first mixture;
(d) measuring the level of a PGHS-mediated arachidonic acid metabolite synthesized in said first mixture;
(e) comparing said amount to the amount of said prostaglandin synthesized by a second portion of said microsomal extract in the presence of arachidonic acid, but in the absence of said compound;
(f) preparing a microsomal extract of a second transgenic mammalian cell line which contains chromosomally integrated, recombinant DNA sequence, wherein said DNA sequence expresses mammalian PGHS-1, and wherein said DNA sequence does not express any mammalian PGHS-2, and wherein said cell line does not express autologous PGHS-2 or PGHS-1 activity;
(g) forming a second aqueous mixture comprising a portion of the microsomal extract of step (f) and a second preselected amount of said compound;
(h) adding arachidonic acid to said mixture of step (g);
(i) measuring the amount of a PGHS-mediated arachidonic acid metabolite synthesized in said mixture of step (g); and
(j) comparing said amount to the amount of said metabolite synthesized by a second portion of said microsomal extract of step (f) in the presence of arachidonic acid, but in the absence of said compound.

10. The method of claim 9 wherein, in step (f), the mammalian PGHS-1 is human PGHS-1.

11. The method of claim 9 or claim 10 wherein, in steps (d) and (i), the metabolite is a prostaglandin.

12. The method of any one of claims 9 to 11 wherein, in steps (a) and (f), the transgenic mammalian cell lines are primate cell lines.

13. An isolated DNA molecule encoding the amino acid sequence of human PGHS-2 wherein said amino acid sequence corresponds to SEQ ID NO. 4.

14. An isolated DNA sequence as claimed in claim 13 wherein the nucleotide sequence corresponds to SEQ ID No. 3.

15. Isolated human PGHS-2 having an amino acid sequence corresponding to SEQ ID No. 4.

16. A transgenic mammalian cell line which contains a chromosomally integrated, recombinant DNA sequence, wherein said DNA sequence encodes human PGHS-2 corresponding to Seq ID No. 4, and wherein said DNA sequence does not express PGHS-1, and wherein said cell line does not express autologous PGHS-1 or PGHS-2 activity.

17. The cell line of claim 16 which is a primate cell line.

18. The cell line of claim 16 which is a murine cell line.

19. The cell line of claim 16 which is a human cell line.

20. The cell line of any one of claims 16 to 19 wherein the recombinant DNA sequence also comprises a promoter.

21. The cell line of any one of claims 16 to 20 wherein the recombinant DNA sequence also comprises a selectable marker gene or a reporter gene.

22. The cell line of any one of claims 15 to 21 wherein the transgenic mammalian cell line is produced by transfection of a mammalian cell line with said recombinant DNA sequence in a plasmid vector, in a viral expression vector or as an isolated DNA sequence.

23. A transgenic primate cell line ATCC CRL 11284.

24. A method for producing human PGHS-2 having a sequence corresponding to SEQ ID No. 4, comprising:
(a) culturing a genetically engineered host-cell comprising a nucleotide sequence encoding human PGHS-2 having a sequence corresponding to SEQ ID No. 4 operatively associated with a heterologous regulatory sequence that controls gene expression so that said human PGHS-2 is stably expressed by said host cells; and
(b) recovering said human PGHS-2 gene product from the cell culture.

## Patentansprüche

1. Verfahren zur Bestimmung der Fähigkeit einer Verbindung zur Inhibierung der durch PGHS-2 katalysierten Prostaglandinsynthese in Säugerzellen, umfassend:
(a) Zugabe einer vorgewählten Menge dieser Verbindung zu einer transgenen Säugerzellinie in Kulturmedium, wobei die Zellinie eine chromosomal integrierte, rekombinante DNA-Sequenz enthält, wobei diese DNA Human-PGHS-2 mit einer SEQ ID Nr. 4 entsprechenden Aminosäuresequenzsequenz exprimiert, und wobei diese DNA PGHS-1 nicht exprimiert, und wobei diese Zellinie keine autologe PGHS-1- oder PGHS-2-Aktivität exprimiert;
(b) Zugabe von Arachidonsäure zu diesem Kulturmedium;
(c) Messung des Spiegels eines von der Zellinie synthetisierten PGHS-vermittelten Arachidonsäuremetaboliten; und
(d) Vergleichen dieses Spiegels mit dem Spiegel dieses Metaboliten, der durch diese Zellinie in Abwesenheit dieser Verbindung synthetisiert wurde.

2. Verfahren nach Anspruch 1, wobei der Metabolit ein Prostaglandin ist.

3. Verfahren zur Bestimmung der Fähigkeit einer Verbindung zur Inhibierung der durch PGHS-2 katalysierten Prostaglandinsynthese, umfassend:
(a) Herstellung eines mikrosomalen Extrakts einer transgenen Säugerzellinie, die eine chromosomal integrierte, rekombinante DNA-Sequenz enthält, wobei diese DNA-Sequenz Human-PGHS-2 mit einer SEQ ID Nr. 4 entsprechenden Aminoisäuresequenz exprimiert, und wobei diese DNA-Sequenz PGHS-1 nicht exprimiert, und wobei diese DNA keine autologe PGHS-1- oder PGHS-2-Aktivität exprimiert;
(b) Herstellung einer gepufferten wäßrigen Mischung, umfassend einen Teil des mikrosomalen Extrakts und eine vorgewählte Menge dieser Verbindung;
(c) Zugabe von Arachidonsäure zu dieser Mischung;
(d) Messung der Menge eines in dieser Mischung synthetisierten PGHS-vermittelten Arachidonsäuremetaboliten; und
(e) Vergleichen dieser Menge mit der Menge dieses Metaboliten, der durch einen zweiten Teil dieses mikrosomalen Extrakts in Gegenwart von Arachidonsäure, aber in Abwesenheit dieser Verbindung synthetisiert wurde.

4. Verfahren nach Anspruch 3, wobei der Metabolit ein Prostaglandin ist.

5. Verfahren zur Bestimmung der Fähigkeit einer Verbindung zur Inhibierung der durch PGHS-2 oder PGHS-1 katalysierten Prostaglandinsynthese in Säugerzellen, umfassend:
(a) Zugabe einer ersten vorgewählten Menge dieser Verbindung zu einer ersten transgenen Säugerzellinie in Kulturmedium, wobei die Zellinie eine chromosomal integrierte, rekombinante DNA-Sequenz enthält, wobei diese DNA-Sequenz Human-PGHS-2 mit einer SEQ ID Nr. 4 entsprechenden Aminoisäuresequenz exprimiert, und wobei diese DNA-Sequenz PGHS-1 nicht exprimiert, und wobei diese Zellinie keine autologe PGHS-1- oder PGHS-2-Aktivität exprimiert;
(b) Zugabe von Arachidonsäure zu dieser Mischung;
(c) Messung des Spiegels eines durch diese erste Zellinie synthetisierten PGHS-vermittelten Arachidonsäuremetaboliten;
(d) Vergleichen dieses Spiegels mit dem Spiegel dieses Metaboliten, der von dieser ersten Zellinie in Abwesenheit dieser Verbindung synthetisiert wurde;
(e) Zugabe einer zweiten vorgewählten Menge dieser Verbindung zu einer zweiten transgenen Säugerzellinie in Kulturmedium, wobei die Zellinie eine chromosomal integrierte, rekombinante DNA-Sequenz enthält, wobei diese DNA-Sequenz Säuger-PGHS-1 exprimiert, und wobei diese DNA-Sequenz kein Säuger-PGHS-2 exprimiert, und wobei diese Zellinie keine autologe PGHS-1- oder PGHS-2-Aktivität exprimiert;
(f) Zugabe von Arachidonsäure zu diesem Kulturmedium;
(g) Messung des Spiegels eines durch diese zweite Zellinie synthetisierten PGHS-vermittelten Arachidonsäuremetaboliten; und
(h) Vergleichen dieses Spiegels mit dem Spiegel dieses Metaboliten, der von dieser zweiten Zellinie in Abwesenheit dieser Verbindung synthetisiert wurde.

6. Verfahren nach Anspruch 5, wobei das Säuger-PGHS-1 in Schritt (e) Human-PGHS-1 ist.

7. Verfahren nach Anspruch 5 oder Anspruch 6, wobei der Metabolit in den Schritten (c) und (g) ein Prostaglandin ist.

8. Verfahren nach einem der Ansprüche 5 bis 7, wobei die transgenen Säugerzellinien in den Schritten (a) und (e) Primatenzellinien sind.

9. Verfahren zur Bestimmung der Fähigkeit einer Verbindung zur Inhibierung der durch PGHS-1 oder PGHS-2 katalysierten Prostaglandinsynthese, umfassend:
(a) Herstellung eines ersten mikrosomalen Extrakts einer ersten transgenen Säugerzellinie, die eine chromosomal integrierte, rekombinante DNA-Sequenz enthält, wobei diese DNA-Sequenz Human-PGHS-2 mit einer SEQ ID Nr. 4 entsprechenden Aminoisäuresequenz exprimiert, und wobei diese DNA-Sequenz PGHS-1 nicht exprimiert, und wobei diese Zellinie keine autologe PGHS-1- oder PGHS-2-Aktivität exprimiert;
(b) Bildung einer ersten wäßrigen Mischung, umfassend einen Teil des ersten mikrosomalen Extrakts und eine erste vorgewählte Menge dieser Verbindung;
(c) Zugabe von Arachidonsäure zu dieser ersten Mischung;
(d) Messung des Spiegels eines in dieser ersten Mischung synthetisierten PGHS-vermittelten Arachidonsäuremetaboliten;
(e) Vergleichen dieser Menge mit der Menge dieses Prostaglandins, die durch einen zweiten Teil dieses mikrosomalen Extrakts in Gegenwart von Arachidonsäure, aber in Abwesenheit dieser Verbindung synthetisiert wurde;
(f) Herstellung eines mikrosomalen Extrakts einer zweiten transgenen Säugerzellinie, die eine chromosomal integrierte, rekombinante DNA-Sequenz enthält, wobei diese DNA-Sequenz Säuger-PGHS-1 exprimiert, und wobei diese DNA-Sequenz kein Säuger-PGHS-2 exprimiert, und wobei diese Zellinie keine autologe PGHS-2- oder PGHS-1-Aktivität exprimiert;
(g) Bildung einer zweiten wäßrigen Mischung, umfassend einen Teil des mikrosomalen Extrakts aus Schritt (f) und eine zweite vorgewählte Menge dieser Verbindung;
(h) Zugabe von Arachidonsäure zu dieser Mischung aus Schritt (g);
(i) Messung der Menge eines in dieser Mischung aus Schritt (g) synthetisierten PGHS-vermittelten Arachidonsäuremetaboliten; und
(j) Vergleichen dieser Menge mit der Menge dieses Metaboliten, die durch einen zweiten Teil dieses mikrosomalen Extrakts aus Schritt (f) in Gegenwart von Arachidonsäure, aber in Abwesenheit dieser Verbindung synthetisiert wurde.

10. Verfahren nach Anspruch 9, wobei das Säuger-PGHS-1 in Schritt (f) Human-PGHS-1 ist.

11. Verfahren nach Anspruch 9 oder Anspruch 10, wobei der Metabolit in den Schritten (d) und (i) ein Prostaglandin ist.

12. Verfahren nach einem der Ansprüche 9 bis 11, wobei die transgenen Säugerzellinien in den Schritten (a) und (f) Primatenzellinien sind.

13. Isoliertes DNA-Molekül, das für die Aminosäuresequenz von Human-PGHS-2 codiert, wobei diese Aminosäuresequenz SEQ ID Nr. 4 entspricht.

14. Isolierte DNA-Sequenz wie in Anspruch 13 beansprucht, wobei die Nucleotidsequenz SEQ ID Nr. 3 entspricht.

15. Isoliertes Human-PGHS-2, das die SEQ ID Nr. 4 entsprechende Aminosäuresequenz aufweist.

16. Transgene Säugerzellinie, die eine chromosomal integrierte, rekombinante DNA-Sequenz enthält, wobei diese DNA-Sequenz für Human-PGHS-2 codiert, das SEQ ID Nr. 4 entspricht, und wobei diese DNA-Sequenz PGHS-1 nicht exprimiert, und wobei diese Zellinie keine autologe PGHS-2-oder PGHS-1-Aktivität exprimiert

17. Zellinie nach Anspruch 16, die eine Primatenzellinie ist.

18. Zellinie nach Anspruch 16, die eine Nagerzellinie ist.

19. Zellinie nach Anspruch 16, die ein Human-Zellinie ist.

20. Zellinie nach einem der Ansprüche 16 bis 19, wobei die rekombinante DNA ebenfalls einen Promotor umfaßt.

21. Zellinie nach einem der Ansprüche 16 bis 20, wobei die rekombinante DNA-Sequenz ebenfalls ein auswählbares Markergen oder ein Reportergen umfaßt.

22. Zellinie nach einem der Ansprüche 15 bis 21, wobei die transgene Säugerzellinie durch Transfektion einer Säugerzellinie mit dieser rekombinanten DNA in einem Plasmidvektor, in einem viralen Expressionsvekor oder als isolierte DNA-Sequenz erzeugt wird.

23. Transgene Primatenzellinie ATCC CRL 11284.

24. Verfahren zur Herstellung von Human-PGHS-2 mit einer SEQ ID Nr. 4 entsprechenden Sequenz, umfassend:
(a) Kultivieren einer gentechnologisch veränderten Wirtszelle, umfassend eine Nucleotidsequenz, die für Human-PGHS-2 mit einer SEQ ID Nr. 4 entsprechenden Sequenz codiert, und die operativ verbunden ist mit einer heterologen Regulationssequenz, die die Genexpression so kontrolliert, daß das Human-PGHS-2 stabil durch diese Wirtszelle exprimiert wird; und
(b) Isolieren dieses Human-PGHS-2-Genprodukts aus der Zellkultur.

## Revendications

1. Procédé de détermination de la capacité d'un ligand à inhiber la synthèse de prostaglandine catalysée par PGHS-2 dans des cellules de mammifère comprenant les étapes consistant à :
(a) ajouter une quantité présélectionnée dudit composé à une lignée cellulaire transgénique de mammifère dans un milieu de culture, ladite lignée cellulaire contenant une séquence d'ADN recombinant intégrée dans les chromosomes, ledit ADN exprimant la PGHS-2 humaine qui a une séquence d'acides aminés correspondant à la séquence SEQ ID n° 4, et ledit ADN n'exprimant pas PGHS-1, et ladite lignée cellulaire n'exprimant pas d'activité autologue PGHS-1 ou PGHS-2 ;
(b) ajouter de l'acide arachidonique audit milieu de culture ;
(c) mesurer le taux de métabolite de l'acide arachidonique médié par PGHS, synthétisé par la lignée cellulaire ; et
(d) comparer ledit taux avec le taux dudit métabolite synthétisé par ladite lignée cellulaire en l'absence dudit composé.

2. Procédé selon la revendication 1 dans lequel le métabolite est une prostaglandine.

3. Procédé de détermination de la capacité d'un composé à inhiber la synthèse de prostaglandine catalysée par PGHS-2, comprenant les étapes consistant à :
(a) préparer un extrait microsomal d'une lignée cellulaire transgénique de mammifère, ladite lignée cellulaire contenant une séquence d'ADN recombinant intégrée dans les chromosomes, ledit ADN exprimant la PGHS-2 humaine qui a une séquence d'acides aminés correspondant à la séquence SEQ ID n° 4, et ledit ADN n'exprimant pas PGHS-1, et ladite lignée cellulaire n'exprimant pas d'activité autologue PGHS-1 ou PGHS-2 ; et
(b) former un mélange aqueux tamponné comprenant une portion de l'extrait microsomal et une quantité présélectionnée dudit composé;
c) ajouter de l'acide arachidonique audit mélange ;
d) mesurer la quantité d'un métabolite de l'acide arachidonique médié par PGHS, synthétisé dans ledit mélange ; et
(e) comparer ladite quantité à ladite quantité dudit métabolite synthétisé par une seconde portion dudit extrait microsomal en présence d'acide arachidonique mais en l'absence dudit composé.

4. Procédé selon la revendication 3 dans lequel ledit métabolite est une prostaglandine.

5. Procédé de détermination de la capacité d'un composé à inhiber la synthèse de prostaglandine catalysée par PGHS-2 ou PGHS-1 dans des cellules de mammifères, comprenant les étapes consistant à :
(a) ajouter une première quantité présélectionnée dudit composé à une première lignée cellulaire transgénique de mammifère dans un milieu de culture, ladite lignée cellulaire contenant une séquence d'ADN recombinant intégrée dans les chromosomes, ledit ADN exprimant la PGHS-2 humaine qui a une séquence d'acides aminés correspondant à la séquence SEQ ID n° 4, et ledit ADN n'exprimant pas PGHS-1, et ladite lignée cellulaire n'exprimant pas d'activité autologue PGHS-1 ou PGHS-2 ;
(b) ajouter de l'acide arachidonique audit milieu de culture ;
(c) mesurer le taux de métabolite de l'acide arachidonique médié par PGHS, synthétisé par ladite première lignée cellulaire ;
(d) comparer ledit taux avec le taux dudit métabolite synthétisé par ladite première lignée cellulaire en l'absence dudit composé ;
(e) ajouter une seconde quantité présélectionnée dudit composé à une seconde lignée cellulaire transgénique de mammifère dans un milieu de culture, ladite lignée cellulaire contenant une séquence d'ADN recombinant intégrée dans les chromosomes, ladite séquence d'ADN exprimant une PGHS-1 de mammifère, et ladite séquence d'ADN n'exprimant pas une PGHS-2 de mammifère, et ladite lignée cellulaire n'exprimant pas d'activité autologue PGHS-1 ou PGHS-2 ;
(f) ajouter de l'acide arachidonique audit milieu de culture ;
(g) mesurer le taux de métabolite d'acide arachidonique médié par PGHS synthétisé par ladite seconde lignée cellulaire ; et
(h) comparer ledit taux au taux dudit métabolite synthétisé par ladite seconde lignée cellulaire en l'absence dudit composé.

6. Procédé selon la revendication 5 dans lequel, dans l'étape (e), la PGHS-1 de mammifère est une PGHS-1 humaine.

7. Procédé selon la revendication 5 ou la revendication 6, dans lequel, dans les étapes (c) et (g), le métabolite est une prostaglandine.

8. Procédé selon l'une quelconque des revendications 5 à 7, dans lequel, dans les étapes (a) et (e), les lignées cellulaires transgéniques de mammifère sont des lignées cellulaires de primates.

9. Procédé de détermination de la capacité d'un composé à inhiber la synthèse de prostaglandine catalysée par PGHS-1 ou PGHS-2 comprenant les étapes consistant à :
(a) préparer un premier extrait microsomal d'une première lignée cellulaire transgénique de mammifère, ladite lignée cellulaire contenant une séquence d'ADN recombinant intégrée dans les chromosomes, ledit ADN exprimant la PGHS-2 humaine qui a une séquence d'acides aminés correspondant à la séquence SEQ ID n° 4, et ledit ADN n'exprimant pas PGHS-1, et ladite lignée cellulaire n'exprimant pas d'activité autologue PGHS-1 ou PGHS-2 ;
(b) former un premier mélange aqueux comprenant une portion du premier extrait microsomal et une première quantité présélectionnée dudit composé ;
c) ajouter de l'acide arachidonique audit premier mélange,
d) mesurer le taux de métabolite d'acide arachidonique médié par PGHS, synthétisé dans ledit premier mélange ;
(e) comparer ladite quantité à la quantité de ladite prostaglandine synthétisée par une seconde portion dudit extrait microsomal en présence d'acide arachidonique mais à l'absence dudit composé ;
(f) préparer un extrait microsomal de la seconde lignée cellulaire transgénique de mammifère, ladite lignée cellulaire contenant une séquence d'ADN recombinant intégrée dans les chromosomes, ladite séquence d'ADN exprimant une PGHS-1 de mammifère, et ladite séquence d'ADN n'exprimant pas une PGHS-2 de mammifère, et ladite lignée cellulaire n'exprimant pas d'activité autologue PGHS-1 ou PGHS-2 ;
(g) former un second mélange aqueux comprenant une portion de l'extrait microsomal de l'étape (f) et une seconde quantité préselectionnée dudit composé ;
(h) ajouter de l'acide arachidonique audit mélange de l'étape (g) ;
(i) mesurer la quantité de métabolite de l'acide arachidonique médié par PGHS, synthétisé dans ledit mélange de l'étape (g) ; et
(j) comparer ladite quantité à la quantité dudit métabolite synthétisé par une seconde portion dudit extrait microsomal de l'étape (f) en présence d'acide arachidonique, mais en l'absence dudit composé.

10. Procédé selon la revendication 9 dans lequel dans l'étape (f) la PGHS-1 de mammifère est une PGHS-1 humaine.

11. Procédé selon la revendication 9 ou la revendication 10 dans lequel, dans les étapes (d) et (i), le métabolite est une prostaglandine.

12. Procédé selon l'une quelconque des revendications 9 à 11, dans lequel, dans les étapes (a) et (f), les lignées cellulaires transgéniques de mammifère sont des lignées cellulaires de primates.

13. Molécule d'ADN isolé codant pour la séquence d'acides aminés de la PGHS-2 humaine, ladite séquence d'acides aminés correspondant à la séquence SEQ ID n° 4.

14. Séquence d'ADN isolé selon la revendication 13, la séquence nucléotidique correspondant à la séquence SEQ ID n° 3.

15. PGHS-2 humaine isolée ayant une séquence d'acides aminés correspondant à la séquence SEQ ID n° 4.

16. Lignée cellulaire transgénique de mammifère qui contient une séquence d'ADN recombinant intégrée dans les chromosomes, ladite séquence ADN exprimant la PGHS-2 humaine correspondant à la séquence SEQ ID n° 4, et ledit ADN n'exprimant pas PGHS-1, et ladite lignée cellulaire n'exprimant pas d'activité autologue PGHS-1 ou PGHS-2.

17. Lignée cellulaire selon la revendication 16 qui est une lignée cellulaire de primate.

18. Lignée cellulaire selon la revendication 16 qui est une lignée cellulaire murine.

19. Lignée cellulaire selon la revendication 16 qui est une lignée cellulaire humaine.

20. Lignée cellulaire selon l'une quelconque des revendications 16 à 19, dans laquelle la séquence d'ADN recombinant comprend en outre un promoteur.

21. Lignée cellulaire selon l'une quelconque des revendications 16 à 20, dans laquelle la séquence d'ADN recombinant comprend en outre un gène marqueur sélectionnable ou un gène rapporteur.

22. Lignée cellulaire selon l'une quelconque des revendications 15 à 21, dans laquelle la lignée cellulaire transgénique de mammifère est produite par transfection d'une lignée cellulaire de mammifère avec ladite séquence d'ADN recombinant dans un vecteur plasmidique, dans un vecteur d'expression viral ou sous la forme d'une séquence d'ADN isolée.

23. Lignée cellulaire transgénique de primate ATCC CRL 11284.

24. Procédé de production de la PGHS-2 humaine ayant une séquence correspondant à la séquence SEQ ID n° 4, comprenant les étapes consistant à :
(a) cultiver une cellule hôte manipulée génétiquement comprenant une séquence nucléotidique codant pour la PGHS-2 humaine ayant une séquence correspondant à la séquence SEQ ID n° 4 associée de manière opérante avec une séquence de régulation hétérologue qui contrôle l'expression des gènes de telle sorte que ladite PGHS-2 humaine soit exprimée de manière stable par lesdites cellules hôtes ; et
(b) recueillir ledit produit du gène de la PGHS-2 humaine à partir de la culture cellulaire.
